# EUROPEAN PATENT APPLICATION

(11) **EP 3 054 412 A1**
(43) Date of publication of application: **10.08.2016**
(21) Application number: 14843220.6
(22) Date of filing: 01.10.2014
(51) Int. Cl.: G06Q 50/22

(54) **HEALTH INFORMATION PROCESSING DEVICE, HEALTH INFORMATION DISPLAY DEVICE, AND METHOD**

(30) Priority: 01.10.2013 JP 2013206834
(71) Applicant: Tohoku University, Sendai-shi, Miyagi 980-8577 (JP); Kabushiki Kaisha Toshiba, Minato-ku Tokyo 105-8001 (JP)
(72) Inventor: NEMOTO, Yasuhisa, Sendai-shi Miyagi 980-8577 (JP); MATSUE, Tomokazu, Sendai-shi Miyagi 980-8577 (JP); TAKAYAMA, Takuzo, Tokyo 105-8001 (JP)
(74) Representative: Moreland, David
(86) International application number: PCT/JP2014/076332
(87) International publication number: WO 2015/050174

(57) **Abstract**

A health information processing apparatus (100) according to an embodiment includes an accumulation unit (110), an analysis unit (121), and an estimation unit (122). The accumulation unit (110) that stores genome information and biological information and behavior information being continuously collected, which are health information of each user, that correspond to a plurality of users. The analysis unit (121) that analyzes the stored health information corresponding to the plurality of users. The estimation unit (122) that estimates a future health risk of a predetermined user by using a result of the analysis and the health information of the predetermined user.

## Description

### Field

An embodiment of the present invention relates to a health information processing apparatus, a health information display apparatus, and methods.

### Background

Conventionally, various countermeasures progress toward the realization of preemptive medicine and individualized prevention in the world. Here, the preemptive medicine represents that, before an outbreak of an illness, the illness is predicted with high accuracy or a diagnosis is made before an outbreak of the illness, and a therapeutic intervention is made at an appropriate time period before the outbreak, and the outbreak is prevented or delayed. In addition, individualized prevention represents prevention of an illness that is suitable to each individual.

However, still, it is difficult to evaluate a healthy state or make a judge of a non-illness state before the arrival at the outbreak of an illness accurately and objectively. For example, while there are many attempts for collecting a life log of an individual and making feedback to the individual, mostly, there is no association with base data necessary for the evaluation of a healthy state or a non-illness state, or individual health guidance based on the individual constitution has not been reached.

### Citation List

### Patent Literature

Patent Literature 1: JP 2001-327472 A
Patent Literature 2: JP 2006-320735 A

### Summary

### Technical Problem

The present invention is devised in consideration of the description presented above, and an object thereof is to provide a health information processing apparatus, a health information display apparatus, and a method capable of performing health guidance of an individual based on the individual constitution by estimating a future health risk with high accuracy.

### Solution to Problem

A health information processing apparatus according to an embodiment includes an accumulation unit, an analysis unit, and an estimation unit. The accumulation unit that stores genome information and biological information and behavior information being continuously collected, which are health information of each user, that correspond to a plurality of users. The analysis unit that analyzes the stored health information corresponding to the plurality of users. The estimation unit that estimates a future health risk of a predetermined user by using a result of the analysis and the health information of the predetermined user.

### Advantageous Effects of Invention

There is an advantage that health guidance of an individual based on the individual constitution can be performed by estimating a future health risk with high accuracy.

### Brief Description of Drawings

FIG. 1 is a diagram that illustrates a motivation-improved society realized by this embodiment.
FIG. 2A is a diagram that illustrates an example of this embodiment.
FIG. 2B is a diagram that illustrates an overview of this embodiment.
FIG. 3 is a diagram that illustrates PHR (Personal Health Record) data according to this embodiment.
FIG. 4 is a diagram that illustrates the collection of life log information according to this embodiment.
FIG. 5 is a diagram that illustrates an analysis of PHR big data according to this embodiment.
FIG. 6 is a diagram that illustrates the type of lifestyle according to this embodiment.
FIG. 7 is a diagram that illustrates a health risk estimation table T according to this embodiment.
FIG. 8 is a diagram that illustrates estimation of a health risk according to this embodiment.
FIG. 9 is a diagram that illustrates a health forecast portal site according to this embodiment.
FIG. 10 is a diagram that illustrates the processing sequence of daily medical checkup according to this embodiment.
FIG. 11 is a diagram that illustrates screen transitions at a portal site for an attending doctor according to this embodiment.
FIG. 12 is a diagram that illustrates screen transitions at a portal site for a user according to this embodiment.
FIG. 13 is a diagram that illustrates a simulation of a health risk according to this embodiment.
FIG. 14 is a diagram that illustrates health risk graphs displayed for an attending doctor and a user in this embodiment.
FIG. 15 is a diagram that illustrates an example (first example) of a secondary use service according to this embodiment.
FIG. 16 is a diagram that illustrates the example (first example) of the secondary use service according to this embodiment.
FIG. 17 is a diagram that illustrates an example (second example) of the secondary use service according to this embodiment.
FIG. 18 is a diagram that illustrates an example (third example) of the secondary use service according to this embodiment.
FIG. 19 is a diagram that illustrates a first one of incentive mechanisms according to this embodiment.
FIG. 20 is a diagram that illustrates a second one of incentive mechanisms according to this embodiment.
FIG. 21A is a diagram that illustrates a comparative analysis between users according to this embodiment.
FIG. 21B is a diagram that illustrates a comparative analysis between users according to this embodiment.
FIG. 22 is a diagram that illustrates a comparative analysis between groups according to this embodiment.
FIG. 23A is a diagram that illustrates a comparative analysis of a predetermined time point of an individual according to this embodiment.
FIG. 23B is a diagram that illustrates the comparative analysis of the predetermined time point of the individual according to this embodiment.
FIG. 24A is a diagram that illustrates a comparative analysis of an individual for a predetermined period according to this embodiment.
FIG. 24B is a diagram that illustrates a comparative analysis of an individual for a predetermined period according to this embodiment.
FIG. 24C is a diagram that illustrates a comparative analysis of an individual for a predetermined period according to this embodiment.
FIG. 25 is a functional block diagram of a PHR processing apparatus according to this embodiment.
FIG. 26 is a diagram that illustrates the hardware configuration of the PHR processing apparatus (or a PHR display apparatus) according to this embodiment. Description of Embodiments

Hereinafter, a health information processing apparatus, a health information display apparatus, and methods according to an embodiment will be described with reference to the drawings. In the embodiment described below, while an example will be described in which each of the health information processing apparatus and the health information display apparatus realizes a plurality of functions (for example, a primary use service, a secondary use service, and the like), the implementation of the plurality of functions is not necessarily an essential configuration. Thus, the health information processing apparatus or the health information display apparatus may be configured to realize some of the plurality of functions.

### (Motivation-improved Society Realized by This Embodiment)

According to the embodiment described below, a motivation-improved society in which a motivation for living is increased is realized. Thus, before description of a specific configuration of the embodiment, first, the realization of the motivation-improved society proposed by us will be described.

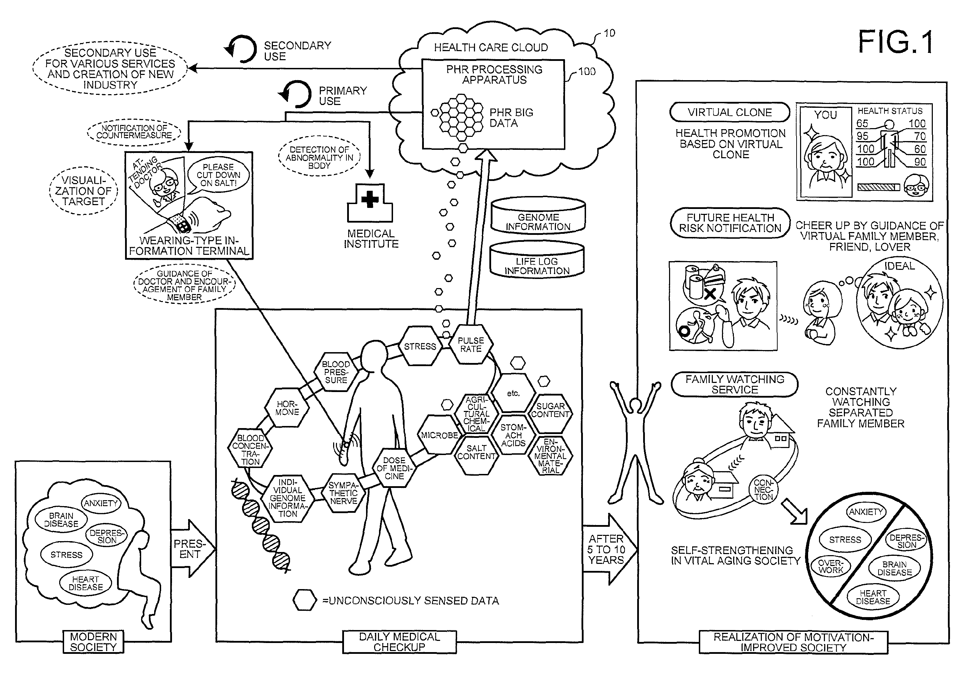

FIG. 1 is a diagram that illustrates the motivation-improved society realized by this embodiment. Nowadays, ideally, everyone lives working and having hobbies healthily and pleasantly in the family and in the region. However, fears for a future illness, dementia, depression, loneliness, worries about separated family members, and the like threaten people in the modern society in which the decreasing birth rate and aging population progress, and a peaceful life has been eroded. In such a situation, far from a future image, even a motivation for living day by day decreases, and a strong mind is not acquired. If there is a tool that can naturally check everyday lives and mental and physical health states of a person and his family members and supports self-actualization drawn through an imagination at any time or a mechanism that raises a will toward efforts for realizing healthy and vital lives of him and his family members in the world, as a result of the self-actualization, everyone increases a motivation for living and is released from the present living, anxiety for the future, or stress and has a strong feeling of easiness and peacefulness. Thus, an economy or a society is recovered in which each individual acquiring such an ideal one's self, being backed by a strong connection with the other family members has vitality and a growing force. Thus, in embodiments described below, by collecting inventive ideas in which semiconductor, communication, energy, material, and medical technologies shine as one, a solution system for recovering a motivation for living was developed and was proposed to be implemented in the society.

As illustrated in FIG. 1, in a 'modern society', anxieties for the health or the living of the future are prevalent, and a connection with the family or the society starts to be lost. While a society of a decreasing birth rate and aging population is impending, people are worried about anxieties, loneliness, and concerns about family members such as being ill, having dementia or depression, being a lonely life, separate family members being well, and how to be confident. The reason for this is that a will or a motivation is started to be lost due to anxieties, depression, stress, a brain diseases, and a heart disease.

In such a 'modern society', everyone ideally desires to live working and having hobbies healthily and pleasantly in the family and in the society. One of means that realizes it is a 'daily medical checkup' illustrated in FIG. 1. This 'daily medical checkup' creates an ideal lifestyle based on innovative PHR (Personal Health Record) big data integrating true vital data in which biological information and behavior information are associated with each other and a constitution database acquired by analyzing genome information of individuals.

In the 'daily medical checkup', this innovative PHR data is collected by using an unconscious sensing technology. As sensing data, as illustrated in FIG. 1, for example, there are a heart rate, stress, blood pressure, a hormone, blood concentration, a sympathetic nerve, the dose of a medicine, and the like. In addition, as the sensing data, for example, there are a sugar content, a salt content, stomach acids, an agricultural chemical, a microbe, an environmental material, and the like. As illustrated in FIG. 1, a PHR processing apparatus 100 is built on a health care cloud 10. The PHR processing apparatus 100 collects and stores biological information and behavior information of each individual with being associated with each other as life log information. Then, the PHR processing apparatus 100, as illustrated in FIG. 1, manages PHR big data acquired by integrating a large amount of the life log information collected in a time series and a constitution database that is based on genome information for a plurality of users in a unified manner on the health care cloud 10.

The PHR processing apparatus 100 analyzes such PHR big data, thereby analyzing a future disease outbreak risk based on genome information, a response and a reaction of a body for an amount of foods, an amount of exercise, or an exercise load, and the like in detail at a high degree. In addition, a design of a daily life targeted for an ideal image such as a selection of a food content, exercise, a lifestyle, and a medicine or a supplement that are optimal to an indication of a disease outbreak risk or an attack, a constitution of an individual, and a lifestyle can be made. Furthermore, the PHR processing apparatus 100, for example, applies big data mining, an integrated genome analysis, a simulation, a visualizing and quantifying technology of communication, and the like.

In this embodiment, the PHR data collected from each individual as above is used not only for a "primary use" for feeding the data back to a corresponding individual in the mechanism of the 'daily medical checkup' but also for a "secondary use" for various services. Thus, hereinafter, regarding the use of the PHR data, in this embodiment, an overview of health care informatics realized on the health care cloud will be described with being divided into the "primary use" and the "secondary use".

First, the mechanism of the 'daily medical checkup' that is the "primary use" will be briefly described. For example, the PHR processing apparatus 100 feeds back a result of the analysis of the PHR big data to a target person by displaying the result to a wearing-type information terminal that is worn by the target person. An example of the feedback is a "future health risk notification". The target person can acquire his future health risk based on the service of the "future health risk notification" provided on the wearing-type information terminal and have a visualized object by receiving a notification of a countermeasure thereof. In addition, the target person can receive guidance from a doctor, an encouragement from a family member (or a virtual family member), or the like on the wearing-type information terminal. For example, in FIG. 1, the target person receives guidance ("Please cut down on salt!") from an attending doctor. In this way, the "future health risk notification" serves also as a response system using an actual person or a virtual person. Thus, according to this embodiment, each individual, based on information that is naturally collected daily with high accuracy, can acquire his health state using the wearing-type information terminal or the like and receive guidance and an encouragement from an attending doctor or a health support staff of the family. In addition, the individual can manage the mental and physical state of him or his family members and check behaviors and lives thereof.

In addition, in the mechanism of the 'daily medical checkup', the PHR processing apparatus 100 may feed such information back not only to the target person but also to a medical institution. For example, a doctor recognizes a disease outbreak reserve of a high risk based on a result of the analysis that is fed back from the PHR processing apparatus 100 and responds to it as is necessary and actively accesses such a person. In addition, the sensing data transmitted from the target person may be helpful for detection of an abnormality of the target person's body. For example, the PHR processing apparatus 100 constantly monitors the sensing data transmitted day after day for a target person of a disease outbreak reserve of a high risk and, when an abnormality is detected among the sensing data, immediately feeds the abnormality to the medical institution.

Regarding the "secondary use", for example, the PHR processing apparatus 100 provides a result of the analysis of the PHR big data for a medical institution, various companies, or the like, thereby contributing to the secondary use for various services and the creation of a new industry. A specific example will be described later.

In this way, as illustrated in FIG. 1, for example, after five to ten years, a motivation-improved society is realized. In FIG. 1, as keywords of technologies used for realizing the motivation-improved society, there are a "virtual clone", a "future health risk notification", and a "family watching service". Among these, the "virtual clone" and the "future health risk notification" are examples of the "primary use". On the other hand, the "family watching service" is an example of the "secondary use".

For example, the PHR processing apparatus 100 sets a "virtual clone" to each target person and realizes a health promotion based on the "virtual clone". For example, the PHR processing apparatus 100 can intuitively display a future image of each target person after X years on which the present life is influenced by presenting a self-image acquired by reflecting a characteristic look predicted from a future health state on the face or the appearance to the target person as a "virtual clone". In addition, an ideal self-image may be set in the "virtual clone". In this embodiment, the "virtual clone" is presented in the "future health risk notification".

In addition, for example, the PHR processing apparatus 100 presents the "future health risk notification" to each target person. In this "future health risk notification", a virtual family member and the virtual self-image (virtual clone) described above are projected. Furthermore, in the "future health risk notification", the degree of a deviation from the person who is ideally designed and his future appearance of a case where the present life is continued are projected, whereby guidance for the ideal is performed. In addition, by having a conversion with a virtual person or family member, a person viewing the "future health risk notification" can receive health guidance by being constantly encouraged or cheered up, a will and vitality are enhanced, and activities and a will toward the self-realization of the ideal can be improved. In other words, a target person can raise his will by receiving guidance from a doctor, a family member (or a virtual family member), a friend (virtual friend), or a lover (virtual lover) through this "future health risk notification". Furthermore, in the "future health risk notification", health can be checked as well.

Furthermore, for example, in this embodiment, as an example of the secondary use service, a "family watching service" is realized. According to this "family watching service", a separated family member can be watched at any time. Ubiquitous life log information can be used as a tool for achieving communication allowing a family member to clearly watch that a separated old person living alone and having proneness to disease has foods and takes medicine well and is in good health, thereby giving a notification of time when the person is not in good condition. As a result, it can be prevented that, since a person has unreasonable patience or performs a stouthearted behavior due to hesitation or worry about his family members, the family members do not recognize the outbreak of an illness to be ignored so as to delay the recognition thereof, and worries and concerns of the family members are also relieved. Thus, bonding with the family members and the society is strengthened, and self-strengthening is achieved in a vital aging society.

In addition, although not illustrated in FIG. 1, according to this embodiment, for example, a will can be improved toward an object of the acquisition of points based on the degree of achievement toward a target for efforts, a function of comparing with a future image of a competing friend, an opening function through an SNS (Social Networking Service) or the like, a function of assigning local currency points as a compensation, and the like. Furthermore, according to this embodiment, the condition of the disease of a person having an illness is watched over, an indication of an attack is detected, and, when the person is not in a good health or in an emergency, a helper or a first-aid staff immediately rushes to care his mind and body.

For example, in a case where anyone can use the "daily medical checkup" described above, he can check and manage the mind and body states, the behaviors, and the statuses of lives of him and his family members, and accordingly, everybody can achieve preemptive medicine and individual prevention that keeps him away from the outbreak of an illness. As a result, his ideal goal is clarified, a will is enhanced toward the realization of sound mind and body, and the feeling of achievement according to the ideal self-realization raises a motivation for the life, and each individual can be strengthened. In addition, according to the "family watching service", the mind and body state, the behavior, and the living status of a separated family member can be acquired, and accordingly, each individual is free from anxieties and worries and constantly feels close bonding between himself and the family member, whereby a secured and peaceful society can be realized. The reason for this is that strong bonding of the family and the society is recovered by supporting daily delicate health maintenance, management of foods and a condition, watching an old person and a child that are not currently sufficient, and the realized society also represents the image of a society of a dream in which a sound, pleasant, secured, and peaceful life can be spent. When such a motivation-improved society is realized, persons are freed from anxieties, depression, stress, brain diseases, and heart diseases that prevail in the modern society.

As described above, the motivation-improved society, in which the "virtual clone", the "future health risk notification", the "family watching service", and the like are provided, realized by using the "daily medical checkup" raises an individual's motivation for living, whereby each individual can be strengthened. In addition, many people use the "daily medical checkup" to accumulate a large amount of PHR big data, and accordingly, it is expected to lead a secondary use for various services and creation of a new industry and allow innovations to be chained in various fields. In this embodiment, a solution system of such a healthcare can be built.

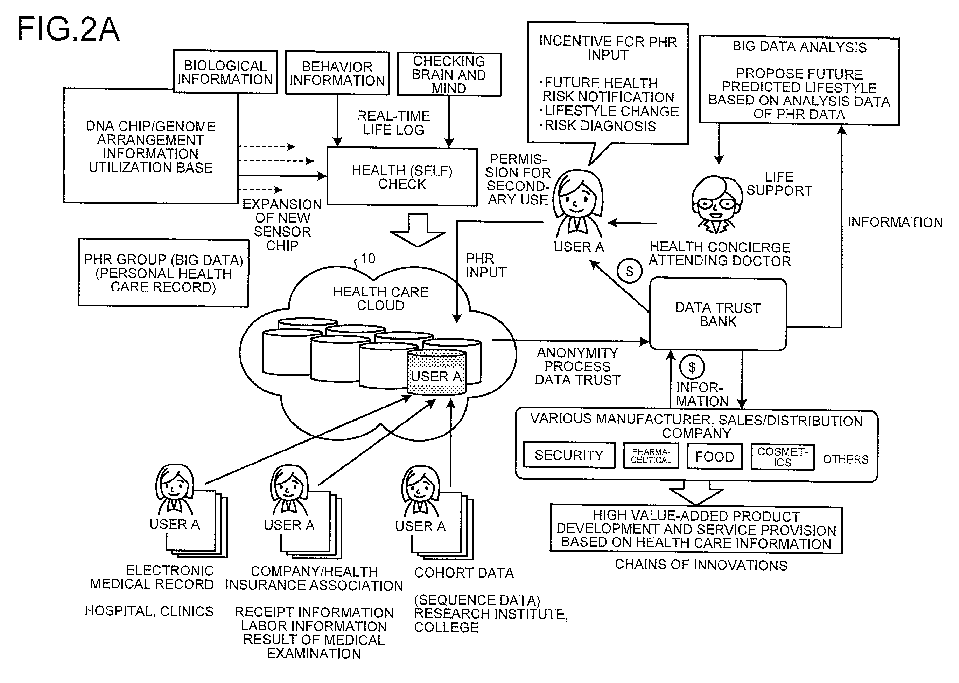

FIG. 2A is a diagram that illustrates an example of this embodiment. As illustrated in FIG. 2A, a solution system according to this embodiment performs health (self) checking based on biological information, which is based on the utilization of DNA chip/genome sequence information, behavior information that is real-time life log, checking of brain and mind, and the like, and information is integrated on the health care cloud 10. For example, for a user A, information of an electronic medical record and the like are integrated from hospitals and clinics. In addition, for user A, receipt information, labor information, results of medical examinations and the like relating to a company and health insurance are integrated. Furthermore, cohort data, sequence data, and the like are integrated from research institutions and colleges. Then, sensing data that is unconsciously collected from user A is integrated ("PHR input" illustrated in the figure).

Such a personal health record (PHR) is managed for each user (for example, user A), and a PHR group in which PHRs of a plurality of persons are integrated is managed in the health care cloud 10 as PHR big data. The PHR big data is operated and managed by a data trust bank (also called a data trust company). For example, the data trust bank enables a future prediction of each individual or a proposal for a lifestyle based on analyzed data of the PHR data by analyzing the PHR big data (big data analysis). For example, an attending doctor who is a health concierge performing a life support proposes a lifestyle based on the analyzed data of the PHR data or provides a "virtual clone" or a "future health risk notification" based on the analyzed data. In other words, for data inputting the PHR data, individual health guidance such as a health forecast on which the constitution and the lifestyle of each individual are reflected, a change in the lifestyle, and a risk diagnosis can be fed back.

Since the above-described feedback can be transferred as an incentive inputting the PHR data, a user continuously inputs the PHR data (the normalization of the PHR data input). In addition, when the user permits the secondary use of the PHR data, the data trust bank can assign a selling right or an access right for the managed PHR data or the analyzed data to various manufacturers or sellers/distributers. Here, since the PHR data and the analyzed data are personal information that requires careful handling, as illustrated in the figure, the anonymity thereof may be set.

As various manufactures or sellers/distributors provided with the PHR data or the analyzed data, for example, there are "security", "pharmaceutical", "food", and "cosmetics" companies, and the various manufacturers or the sellers/distributors can perform development of high value-added products or provision of services based on health care information such as the PHR data or the analyzed data that has been provided. Here, the development of products or services, which is performed by various manufactures or the sellers/distributors, is over a very broad field including a clinical test performed for the development of medical and pharmaceutical products or approvals defined in the pharmaceutical affairs law and simple marketing for collecting biological information appearing in the bodies from viewers of a movie or a program. The solution system of this embodiment can generate chained innovations in each field by being used in such a broad field.

In addition, as each individual uses the "daily medical checkup", the solution system of this embodiment can build and provide a new sensor for the individual (for example, a sensor optimal to the person is provided based on the genome information or the lifestyle information of the user) or promote the development of a new DNA chip based on the analyzed data of the PHR data.

### (Overview of This Embodiment)

In this embodiment, first, a large-scale genome cohort database 114a is formed by integrating the PHR data including the genome information on the health care cloud 10, and, by setting the PHR big data accumulated in this large-scale genome cohort database 114a as base data, a mechanism for estimating a future health risk (for example, the probability of the outbreak of each disease) with high accuracy is built. In addition, by continuously collecting the PHR data of each individual from each field and managing the collected data in a unified manner, for the individual, a mechanism (the daily medical checkup) is built which feeds back individual health guidance on which the constitution and the lifestyle of the individual is reflected. Furthermore, the mechanism of the secondary use (a use for the other people or a commercial use) of the PHR big data integrated on the health care cloud 10.

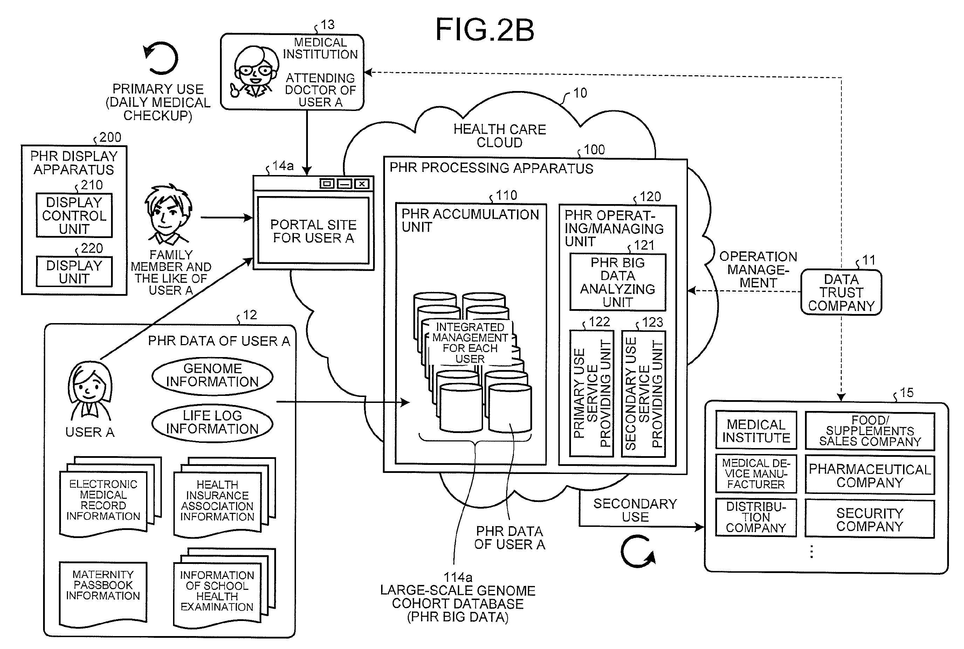

FIG. 2B is a diagram that illustrates an overview of this embodiment. As illustrated in FIG. 2B, the PHR processing apparatus 100 (also referred to as a "health information processing apparatus") is built on the health care cloud 10, and the PHR processing apparatus 100 realizes the various mechanisms described above. In addition, as illustrated in FIGS. 2B, a health care cloud service including the operation of the PHR processing apparatus 100 is operated and managed by a data trust company 11. For example, the data trust company 11 performs various procedures for providing services in an online or offline mode for a user and a medical institution 13 provided with the primary use service (the daily medical checkup) and a medical institution and various companies 15 provided with the secondary use service (see a dotted line illustrated in FIG. 2B).

The PHR processing apparatus 100 includes: a PHR accumulation unit 110 that collects and accumulates PHR data; and a PHR operating/managing unit 120 that operates and manages the PHR data accumulated in the PHR accumulation unit 110.

The PHR accumulation unit 110 collects PHR data (the PHR data 12 illustrated in FIG. 2B) relating to an individual from not only individuals but also from a research institution, a medical institution, a company, and the like, integrates the collected data as the PHR data of each individual, and individually manages the integrated data in a unified manner. For example, as the PHR data, in addition to information of a life log that is continuously collected from an individual, there are genome information of the individual that is acquired from a research institution, electronic medical record information that is acquired from a medical institution, health insurance association information (receipt information, labor information, and examination notebook information) acquired from a company or a health insurance association, maternity passbook information, information of a school health examination, and the like. In other words, the PHR data is collected not only from an individual but also from various organizations as information relating to the health of the individual, and the kind thereof is not particularly limited. In addition, the PHR accumulation unit 110 collects such PHR data in a large scale (for example, a scale of 150,000 persons) and forms a large-scale genome cohort database 114a. In the large-scale genome cohort database 114a, the scale is enlarged by accumulating new information on a daily basis for each individual, and the scale is enlarged by enlarging the range of collection targets. Hereinafter, in a case where the entire PHR data of the large-scale genome cohort database 114a is to be represented, it will be referred to as "PHR big data" so as to be discriminated from the PHR data of each individual. The PHR data may be also referred to as "health information".

The PHR operating/managing unit 120 includes: a PHR big data analyzing unit 121; a primary use service providing unit 122 (also referred to as an "estimation unit"); and a secondary use service providing unit 123 (also referred to as an "output unit"). The PHR big data analyzing unit 121 analyzes the PHR big data accumulated in the large-scale genome cohort database 114a according to a predetermined object, thereby deriving relevance among the genome information, the lifestyle, and the health risk. Then, the PHR big data analyzing unit 121 acquires an analysis result in which specific relevance with a combination of the constitution of an individual and a combination of lifestyles is represented.

For example, the PHR big data analyzing unit 121 performs a cohort analysis of the PHR big data as a target, thereby deriving relevance between a combination of the type of genome and the type of lifestyle and a risk (it will be referred to as a "disease outbreak risk") of a disease that may be developed in the future. Then, the primary use service providing unit 122 applies the relevance derived by the PHR big data analyzing unit 121 to the PHR data of each individual, thereby calculating a disease outbreak risk according to the constitution of the individual and the lifestyle. Then, the primary use service providing unit 122 performs registration of information of the calculated disease outbreak risk in a portal site 14a of the user or the like, thereby feeding the information back to the user. This portal site 14a can be read by not only the corresponding individual but also his family members and attending doctor, and communication with a third party can be achieved through the portal site 14a. This is the overview of the "daily medical checkup" according to this embodiment. The "daily medical checkup" will be described later in detail.

In addition, the PHR big data analyzing unit 121 performs the cohort analysis of the PHR big data as a target, thereby deriving an analysis result for the secondary use service. In addition, the secondary use service providing unit 123 outputs the analysis result derived by the PHR big data analyzing unit 121, thereby providing the analysis result for various companies (a medical institution, a food/supplements seller, a pharmaceutical company, a medical device manufacturer, a distribution company, a security company, or the like). A specific example of the secondary use service will be described later.

In addition, as illustrated in FIG. 2B, the user, related persons such as user's family members, and the user's attending doctor, for example, read the portal site 14a provided by the primary use service providing unit 122 by using a PHR display apparatus 200 (also referred to as a "health information display device"). The PHR display apparatus 200 is a smartphone, a PC (Personal Computer), an Internet TV, a wearable information terminal, or the like. The PHR display apparatus 200 includes a display control unit 210 and a display unit 220. The display control unit 210 displays the future health risk of the user on the display unit 220.

### (PHR Data)

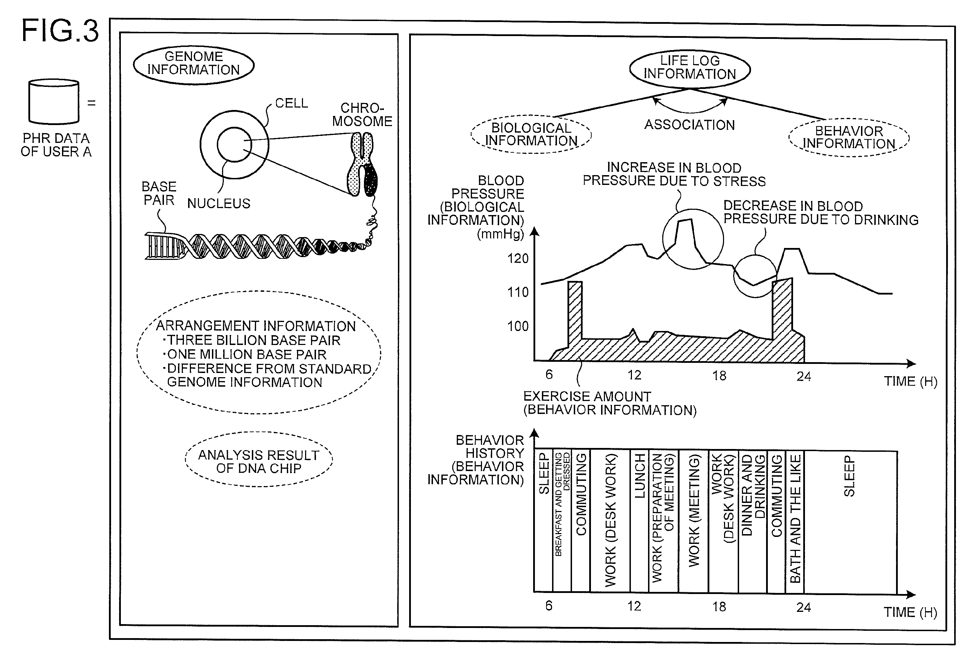

Next, FIG. 3 is a diagram that illustrates the PHR data according to this embodiment. As described above, the PHR data is collected not only from an individual but also from various organizations as information relating to the health of the individual, and the type thereof is not particularly limited. Thus, in this embodiment, information to be collected as the PHR data is considered to be different for each individual. As will be described below, the "daily medical checkup" according to this embodiment calculates the type of lifestyle of an individual based on the PHR data of the individual. In this embodiment, the type of lifestyle is calculated by evaluating ten items (smoking, drinking, sleep, stress, exercise, a food life, a medicine/supplement, a mental state, fatigue, and immunity). Accordingly, in this embodiment, it is assumed that the PHR data from which the ten items can be evaluated is collected from each individual. In FIG. 3, among the PHR data of user A, only the genome information and the life log information will be conceptually illustrated.

First, the genome information is genetic information of user A. As illustrated in FIG. 3, inside a nucleus of a cell, a chromosome is present, and a material composing this chromosome called deoxyribonucleic acid is DNA. DNA has a double helical structure, in which nucleotides that are constitutional units thereof are bonded in a chained pattern, formed by two chains. In addition, a gene is a partition on the DNA. In the nucleotide, deoxyribose sugar is connected using phosphoric acid, and one of four kinds of bases is combined with the deoxyribose sugar. Between the two chains, a base pair of adenine (A) and thymine (T) and a base pair of guanine (G) and cytocine (C) are combined. Human genome information is composed by about three billion base pairs.

In this embodiment, the genome information is arrangement information of these base pairs of about three billion or arrangement information of about one million base pairs determining the individuality. In addition, the PHR accumulation unit 110 may directly store the arrangement information of the base pairs or may store the arrangement information in the form of a difference from standard genome information (for example, an SNP (Single Nucleotide Polymorphism) of a Japanese person). For example, in a case where a user A provides his blood to a research institution, and all the base arrangements (arrangement information) of the genome extracted from user A are specified in the research institution, the arrangement information is handled as the genome information of user A.

In addition, the genome information is not limited to the above-described arrangement information but also includes an analysis result according to various techniques such as a DNA chip and the like. For example, in a case where user A provides his blood to a research institution, and the research institution analyzes the blood using the DNA chip, the analysis result thereof is handled as the genome information of user A. For example, in a case where the type of gene relating to a specific disease (for example, hypertension, hyperlipidemia, obesity, diabetes, or the like), metabolism of a specific medicine, or the type of gene relating to alcohol decomposition is determined, for example, through an SNP analysis using a DNA chip, a CNV (Copy Number Variation) analysis, a micro satellite analysis, an epigenome analysis, a gene expression level analysis, or the like, the analysis result thereof is the genome information of user A.

Next, the life log information is information that represents the lifestyle (mode of life) of user A. As illustrated in FIG. 3, in this embodiment, the biological information and the behavior information will be referred altogether to as the life log information, and both are associated with each other as is necessary, whereby accurate life log information can be acquired.

Here, the acquisition of accurate life log information, which is acquired by associating the "blood pressure" that is the biological information and the "amount of exercise" and a "behavior history" that are the behavior information will be described with reference to FIG. 3. For example, it is assumed that user A wears a biological sensor and an acceleration sensor. Such sensors may be provided in a wearing-type information terminal to be described later or may be installed (for example, attached) to user A separated from the wearing-type information terminal. The biological sensor, for example, detects a change in the blood flow of a peripheral blood vessel of a finger, a wrist, an ear, or the like and acquires blood pressure, a pulse rate, a pulse count, and the like based on the detected change in the blood flow. On the other hand, the acceleration sensor detects the user's posture based on a DC component and identifies a user's operation (walking, running, riding on a bicycle, moving on a vehicle, moving by subway, or the like) based on an AC component. Then, the acceleration sensor acquires the amount of user's exercise based on the user's posture and the user's operation. In addition, the behavior history is acquired from schedule information that is input from a wearing-type information terminal or an information terminal such as a smartphone or a PC.

Then, in this embodiment, the biological information acquired from the biological sensor and the behavior information acquired from the acceleration sensor and the other information terminals are associated with each other by using time information included in each information or are associated with each other by being recorded in the same record. By referring back to FIG. 3, for example, while the blood pressure that is the biological information temporarily rises during the day, by referring to the behavior history associated with the biological information, this rise is determined to be caused by stress due to meetings. In addition, while the same blood pressure falls after the evening, by referring to the behavior history associated with the biological information, this fall is determined to be caused by drinking. Furthermore, relevance between the blood pressure and the amount of exercise becomes clear.

As above, in this embodiment, the biological information and the behavior information are basically handled with being associated with each other. In addition, in this embodiment, the PHR processing apparatus 100 appropriately selects the type of lifestyle and information required for evaluating the health state of the present as biological information and behavior information to be selected. For example, the biological information is information of various numerical values representing the health state of the present and information representing the amount of a component taken into the body and presence/no-presence of a material. For example, the biological information is blood pressure, a heart rate, a pulse count, a body temperature, a body component, ions, pH concentration, and the like. In addition, for example, the biological information is the amounts of components such as sugar, salt, and the like, the concentration of gastric acid, presence/no-presence of agrochemical, an environmental material, and a food additive, the intake amounts of alcohol, nicotine, and a medicament component, and the like. Furthermore, the behavior information is an amount of exercise, sleeping hours, schedule information, positional information of a GPS (Global Positioning System) or the like, and the like. In this embodiment, the whole or a part of such life log information is collected from sensors or various information terminals. In addition, for example, information acquired by a smartphone (a motor system application, a scheduling application, or the like), an SNS, an electronic receipt, or the like may be used.

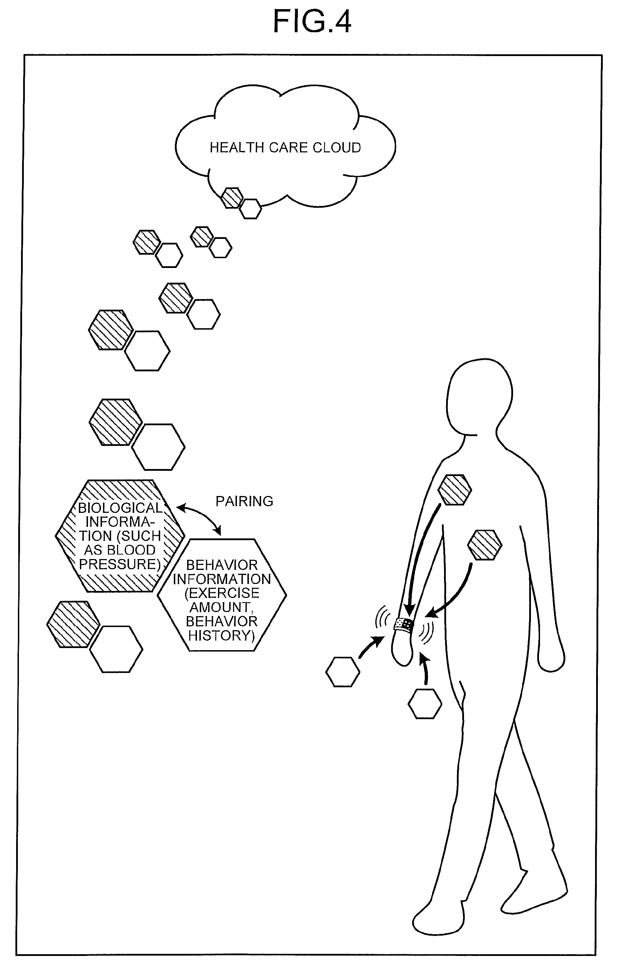

FIG. 4 is a diagram that illustrates the collection of the life log information according to this embodiment. In this embodiment, as an example, it is assumed that a user wears a wearing-type information terminal. As the wearing-type information terminal, for example, a wrist watch type, a glass type, a ring type, or the like may be considered. This wearing-type information terminal has a function of a sensor and can collect biological information. In addition, this wearing-type information terminal also has a function of a so-called information terminal and can collect behavior information. Thus, the wearing-type information terminal achieves the role of a dock of the life log information and, as illustrated in FIG. 4, associates (pairs) the biological information and the behavior information, which have been individually collected from a user, with each other and uploads the life log information after the pairing into the health care cloud 10. While the biological information and the behavior information, which are individually collected, are collected regularly or irregularly during one day, the upload thereof into the health care cloud 10, for example, is performed at the frequency of once per day. In addition, this wearing-type information terminal may receive the biological information and the behavior information from a sensor or an information terminal that is worn by the user separately from the wearing-type information terminal. Also in such a case, the wearing-type information terminal associates (pairing) the biological information and the behavior information, which are individually collected from the user, with each other and uploads the life log information after the pairing into the health care cloud 10. In addition, the paring process may be performed not on the side of the wearing-type information terminal but on the side of the health care cloud 10.

In addition, in this embodiment, the wearing-type information terminal performs personal authentication. In other words, the wearing-type information terminal performs personal authentication for checking whether or not a person wearing the terminal is reliably authenticated. For example, in the case of the wrist watch type, the wearing-type information terminal performs personal authentication through vein authentication. In addition, for example, in a case where a camera is included, the wearing-type information terminal performs personal authentication through face authentication. Furthermore, for example, in the case of the glass type, the wearing-type information terminal performs personal authentication through retina authentication or iris authentication. In addition, for example, in the case of a ring type, the wearing-type information terminal performs personal authentication through vein authentication of the finger. Here, a technique used for the personal authentication is not limited to the techniques described above. In this embodiment, while the technique for uploading the life log information from the wearing-type information terminal has been described, the embodiment is not limited thereto. For example, the life log information may be uploaded from a mobile-type information terminal or an installation-type information terminal.

### (Analysis of PHR Big Data and Estimation of Health Risk Using Analysis Result)

Subsequently, the cohort analysis that is performed for the PHR big data of the large-scale genome cohort database 114a as a target will be described. Here, as described above, in this embodiment, in order to perform the evaluation of the health state and the estimation of the health risk with high accuracy, the large-scale genome cohort database 114a is formed, and the database is set as base data. For example, in the cohort analysis to be described later, the PHR big data analyzing unit 121, in lifetime PHR data from birth to death, associates the outbreak of a disease to a clinical outcome with information of the life and the environment at that time. In addition, in the cohort analysis to be described later, for example, the PHR big data analyzing unit 121 performs a long-term follow-up survey for a cohort of a specific region, performs a comparative analysis with a cohort of another region, and reviews a difference between regions. Such an analysis can be realized by having the large-scale genome cohort database 114a as its target, and, in the case of a small-scale database, the analysis cannot be realized and is limited to an analysis having a specific disease or the like as its target. In addition, in this embodiment, since the life log information included in the PHR big data is collected by using a sensing technology or the like, an accurate and precise analysis can be performed differently from a reply in a conventional interview. Furthermore, by forming the large-scale genome cohort database 114a, a low-frequency allege of a Japanese can be acquired, a comprehensive Japanese original standard SNP database can be built, and a typing array can be standardized.

In this embodiment, the PHR big data analyzing unit 121 performs a cohort analysis of the PHR big data accumulated in the large-scale genome cohort database 114a as its target and derives relevance between a combination of the type of genome and the type of lifestyle and a health risk (in other words, a disease outbreak risk).

Here, in this embodiment, the cohort analysis is a technique for deriving relevance between a factor (a group matching a specific combination of a type of genome and a type of lifestyle) and a disease outbreak by tracking a group (a group matching a specific combination of a type of genome and a type of lifestyle) exposed to a specific factor and a group (a group not matching the combination) not exposed to the factor for a predetermined period and comparing outbreak probabilities of a specific disease with each other. For example, the PHR big data analyzing unit 121 classifies in patterns standard data of a healthy person that is stored in the large-scale genome cohort database 114a, deviation data between a healthy person and a potential sick person, deviation data between a healthy person and a person developing a symptom, an abnormality sign in the life log information, and the like and clarifies relevance with the type of genome. Here, the technique used for the analysis by the PHR big data analyzing unit 121 is not limited to the cohort analysis described above, but any other technique may be used.

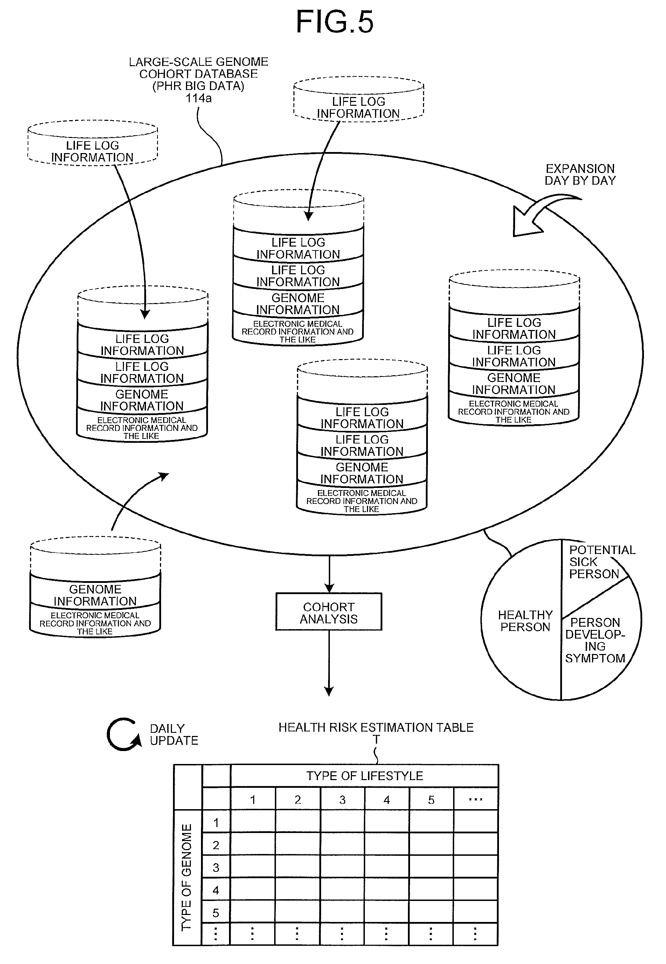

FIG. 5 is a diagram that illustrates the analysis of PHR big data according to this embodiment. As illustrated in FIG. 5, in the large-scale genome cohort database 114a, the life log information that is the PHR data of each individual and the like are newly accumulated day by day, and the PHR data of a new individual is accumulated as a new operating/managing target, whereby the scale of the large-scale genome cohort database 114a increases day by day. In addition, in this large-scale genome cohort database 114a, for example, since the PHR data of the lifetime of each individual is accumulated, from a different viewpoint, the PHR data of healthy persons, potential sick persons, and persons developing symptoms is accumulated.

As illustrated in FIG. 5, the PHR big data analyzing unit 121 performs a cohort analysis of this large-scale genome cohort database 114a as a target and generates a "health risk estimation table T" used for estimating a health risk for each combination of the type of genome and the type of lifestyle. In addition, as described above, the PHR accumulation unit 110 newly accumulates the PHR data, thereby increasing the scale of the large-scale genome cohort database 114a. Thus, the PHR big data analyzing unit 121 newly performs an analysis according to a daily increase in the large-scale genome cohort database 114a, thereby acquiring the "health risk estimation table T" that is a new analysis result. The primary use service providing unit 122 estimates a health risk by using the analysis result that is newly acquired. Accordingly, the accuracy of the "health risk estimation table T" is improved day by day, and the accuracy of the estimation of the health risk that is made by the primary use service providing unit 122 is also improved day by day.

First, in this embodiment, the PHR big data analyzing unit 121 sets the combination pattern of a base pair or a plurality of base pairs among three billion base pairs or the combination pattern of a base pair or a plurality of base pairs among one million base pairs regarded to represent the individuality of a person as the type of genome.

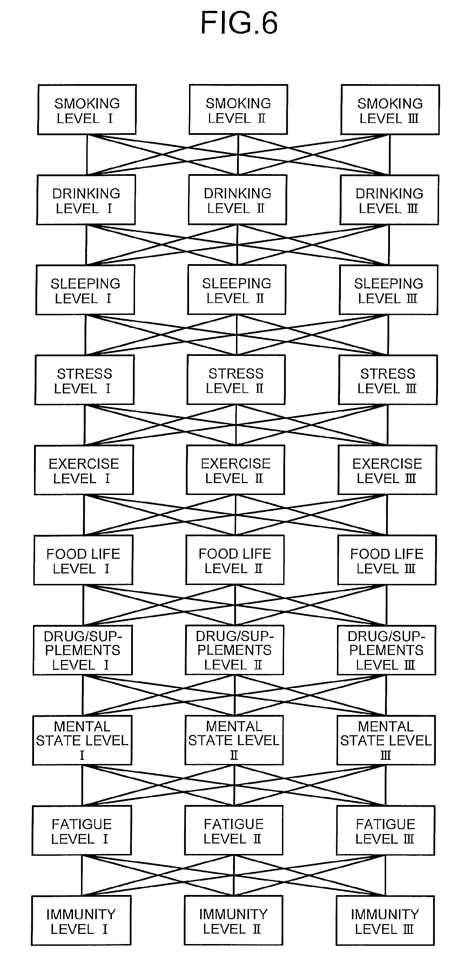

FIG. 6 is a diagram that illustrates the type of lifestyle according to this embodiment. As illustrated in FIG. 6, the PHR big data analyzing unit 121 classifies ten items acquired from the life log information respectively into three levels of "level I" to "level III" and sets patterns of all the combinations thereof (for example, combinations corresponding to tenth power of three) as the types of lifestyles. In this embodiment, the types of lifestyles are merely examples, and the items and the levels may be arbitrary changed. In addition, the method of deriving the type of lifestyle may be arbitrarily changed.

Accordingly, while the number of combinations of a type of genome and a type of lifestyle is a large number, at the beginning, combinations of types of which the relevance with the outbreak of a disease is cleared by the cohort analysis performed by the PHR big data analyzing unit 121 are considered to be some thereof. As a daily increase in the large-scale genome cohort database 114a, outcomes of researches that individually progress, and the like are gradually reflected, the number of combinations of the types of which the relevance with the outbreak of a disease is cleared gradually increases, and blank fields located inside the health risk estimation table T are gradually filled up reflecting the result.

In each cohort analysis process, the PHR big data analyzing unit 121 maintains an algorithm for deriving the ten items based on the life log information in advance. For example, the PHR big data analyzing unit 121 derives smoking/non-smoking of the user and a smoking level representing the degree of smoking based on the "intake amount of nicotine" that is acquired from the biological sensor as the biological information. In addition, the PHR big data analyzing unit 121 derives drinking/no-drinking of the user and a drinking level representing the degree of drinking based on the "intake amount of alcohol" that is acquired from the biological sensor as the biological information. Furthermore, for example, the PHR big data analyzing unit 121 derives a sleeping level such as a user's sleeping time and a sleeping quality from the "heart rate" acquired from a sensor as the biological information, "time at which an alarm has been set" and "alarm time" acquired from a smartphone as the behavior information, and a living sound acquired from a sensor.

In addition, for example, the PHR big data analyzing unit 121 derives a stress level representing the degree of stress felt by the user from the "blood pressure" and the "heart rate" acquired from sensors as the biological information, the "schedule information" acquired from a smartphone as the behavior information, and the like. In addition, for example, the PHR big data analyzing unit 121 derives an exercise level representing the degree of exercise performed by the user from the "heart rate" acquired from a sensor as the biological information, the posture and the operation of the user that are acquired from sensors as the behavior information, the "exercise information" acquired by the motor system application of a smartphone as the behavior information, and the like. Furthermore, for example, by measuring the balance between a sympathetic nerve and a parasympathetic nerve from a change in the peripheral body temperature or the degree of perspiration that is measured by a sensor, the degree of strain and relaxation of the mind is derived. In addition, for example, the PHR big data analyzing unit 121 derives a level of eating habits that represents the user's eating habits from the "sugar content", the "salt content", the "gastric acid", the "alcohol intake amount" and the like acquired from sensors as the biological information. In addition, for example, the PHR big data analyzing unit 121 derives a level of pharmaceutical supplements representing a medicine and a supplement that are taken by the user from the "medicament component" and the like that are acquired from sensors as the biological information. The above-described algorithm is merely an example.

In this way, the PHR big data analyzing unit 121 acquires values of the ten items described above from one side of the biological information and the behavior information included in the life log information or a combination of both sides and derives the level of each item based on these values. Here, for the same target person, while the type of genome is basically not changed, the type of the lifestyle may change in accordance with an elapse of time.

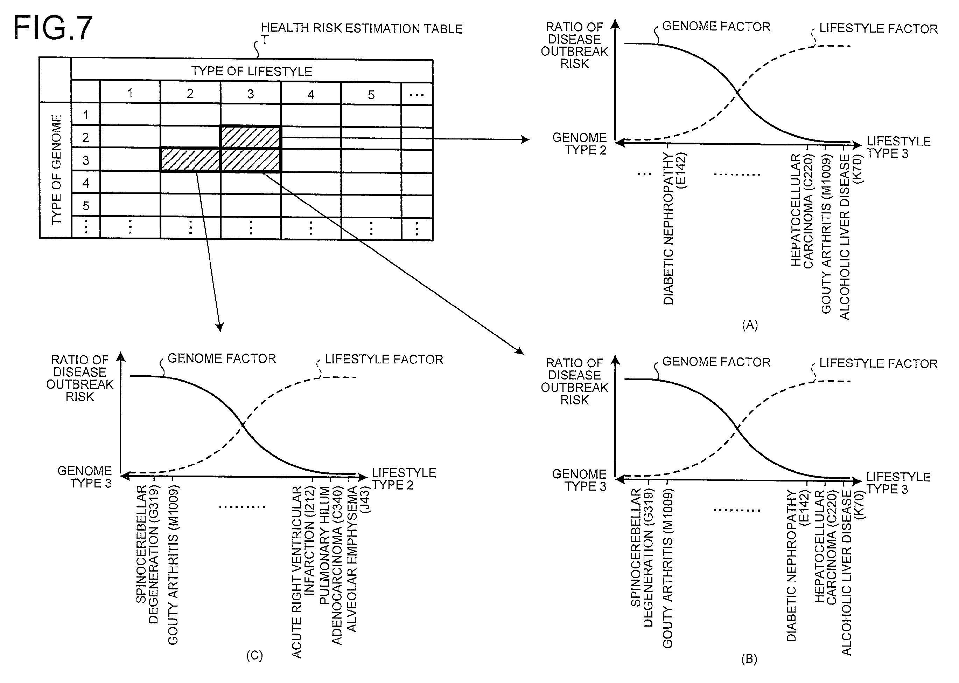

FIG. 7 is a diagram that illustrates the health risk estimation table T according to this embodiment. In this embodiment, it is assumed that, even for users having the same type of lifestyle, in a case where the types of genome are different from each other, the kinds and the order of diseases having high outbreak risks are different from each other. In addition, even for users having the same type of genome, in a case where the types of lifestyles are different from each other, the kinds and the order of diseases having high outbreak risks are different from each other. A method of representing the health risk estimation table T illustrated in FIG. 7 is merely an example, and also the kinds and the order of diseases illustrated in FIG. 7 are merely an example used for the convenience of description.

For example, the PHR big data analyzing unit 121 generates a health risk graph representing a disease outbreak risk for each combination of a type of genome and a type of lifestyle. In each health risk graph, the vertical axis represents ratios of a lifestyle factor and a genome factor in the disease outbreak risk, and diseases are aligned in the horizontal axis. As a disease is located to the further right side in the horizontal axis, it represents that the disease has a strong influence of the lifestyle factor. On the other hand, as a disease is located to the further left side, it represents that the disease has a strong influence of the genome factor. In other words, the health risk graph is a list of diseases, which can be caused in the future, ordered according to a stronger influence of either the genome factor or the lifestyle factor for each combination of a type of genome and a type of lifestyle. In addition, in the horizontal axis, an official name of a disease as a name of the disease, and an ICD (International Classification of Diseases) code that is based on the international classification of diseases are displayed. However, this embodiment is not limited thereto, and for example, only one of the official name and the ICD code of the disease may be displayed.

For example, when (A) and (B) of FIG. 7 are compared with each other, even for users having the same lifestyle type 3, in a case where the types of genome are different as being type 2 and type 3, it can be understood that the kinds and the order of diseases having high outbreak risks are different. For example, while "alcoholic liver disease (K70)" is commonly a disease that is strongly influenced by the lifestyle factor, "gouty arthritis (M1009)" that is a disease strongly influenced by the lifestyle factor for users of genome type 2 is placed as a disease that is strongly influenced rather by the genome factor for users of genome type 3. On the contrary, "diabetic nephropathy (E142)" that is a disease strongly influenced by the lifestyle factor for users of genome type 3 is placed as a disease that is strongly influenced rather by the genome factor for users of genome type 2.

For example, when (B) and (C) of FIG. 7 are compared with each other, even for users having the same genome type 3, in a case where the types of lifestyle are different as being type 3 and type 2, it can be understood that the kinds and the order of diseases having high outbreak risks are different. For example, while "alcoholic liver disease (K70)", "hepatocellular carcinoma (C220)", and "diabetic nephropathy (E142)" are placed as diseases that are strongly influenced by the lifestyle factor for users of lifestyle type 3, for users of lifestyle type 2, "alveolar emphysema (J43)", "pulmonary hilum adenocarcinoma (C340)", "acute right ventricular infarction (1212)", and the like are placed as diseases that are strongly influenced by the lifestyle factor. For example, a case in which, among users of the same genome type 3, the lifestyle type 3 is a user having a high drinking level, and the lifestyle type 2 is a user having a high smoking level or the like may be considered. In addition, for users of the same genome type 3, regardless of the type of lifestyle, "spinocerebellar degeneration (G319)", "gouty arthritis (M1009)", and the like are positioned as diseases that are strongly influenced by the genome factor.

Here, an example of the process of generating the "health risk graph" that is performed by the PHR big data analyzing unit 121 will be described. As a specific example, a case will be described in which the "health risk graph" is generated for a combination of the genome type 3 and the lifestyle type 3.

For example, the PHR big data analyzing unit 121 specifies "disease A, disease B, disease C, and disease D" as diseases having high outbreak risks for users of the genome type 3 by referring to clinical history information (for example, it can be acquired from the electronic medical record information) of users having the genome type 3 as the genome information. In addition, the PHR big data analyzing unit 121 specifies "disease D, disease E, disease F, and disease G" as diseases having high outbreak risks for users of the lifestyle type 3 by referring to clinical history information of users having the lifestyle type 3 as the life log information. Then, the PHR big data analyzing unit 121 compares the specified diseases with each other and classifies the "disease A, disease B, and disease C" that are included only in the diseases having high outbreak risks into "diseases having a strong influence of the genetic factor" for users of the genome type 3. In addition, the PHR big data analyzing unit 121 classifies the "disease E, disease F, and disease G" that are included only in the diseases having high outbreak risks into "diseases having a strong influence of the lifestyle factor" for users of the lifestyle type 3. Furthermore, the PHR big data analyzing unit 121 classifies the "disease D" included in both thereof into a "disease having strong influences of the lifestyle factor and the genetic factor".

Subsequently, the PHR big data analyzing unit 121 specifies diseases having high outbreak risks for users of a combination of genome type 3 and lifestyle type 3 by referring to the clinical history information of users of the combination of genome type 3 and lifestyle type 3. Here, for example, the PHR big data analyzing unit 121 is assumed to specify "disease A, disease C, disease F, and disease G" as diseases having high outbreak risks for users of the combination of genome type 3 and lifestyle type 3. In such a case, the PHR big data analyzing unit 121 determines "disease A" and "disease C" that are common to "disease A, disease B, and disease C" classified into "diseases having strong influences of the genetic factor" in advance as "diseases having strong influences of the genetic factor" and positions the determined diseases on the left side on the "health risk graph" illustrated in FIG. 7 in the horizontal axis. In addition, the PHR big data analyzing unit 121 determines "disease F" and "disease G" that are common to "disease E, disease F, and disease G" classified into "diseases having strong influences of the lifestyle factor" in advance as "diseases having strong influences of the lifestyle factor" and positions the determined diseases on the right side on the "health risk graph" illustrated in FIG. 7 in the horizontal axis.

The PHR big data analyzing unit 121 generates the health risk estimation table T illustrated in FIG. 7 under a specific criterion. For example, the PHR big data analyzing unit 121 generates the health risk estimation table T under a criterion of "a health risk (an outbreak probability of 30%) after 10 years of a case where a person who is in a standard health state continues living of the same lifestyle type, for example, for one year. Regarding this point, generally, the lifestyle type of an actual user is regarded to be different according to the length of the period such as one day, one week, one month, or one year. For example, there may be a case where, while the amount of drinking has increased particularly due to many welcome and farewell parities, the amount of drinking is not that much in terms of one month. Thus, when a user's health risk is to be estimated by using the health risk estimation table T, the primary use service providing unit 122 performs individual estimation according to the period (referred to as an estimation target period) of the PHR data used for the estimation and adjustment according to the health state of the present. In addition, the PHR big data analyzing unit 121 may appropriately change the criterion described above. Furthermore, the PHR big data analyzing unit 121 may set a plurality of future "time points" for the estimation among criteria described above (for example, after one day, after one week, after one month, after one year, after five years, after 10 years, after 20 years, or the like). In such a case, the PHR big data analyzing unit 121 generates a health risk estimation table T corresponding to each criterion. In addition, when the health risk estimation tables T of mutually-different "time points" are compared with each other, for example, there are cases where diseases that are immediately caused are listed in the health risk estimation table in the health risk estimation table T after one month, and diseases that is caused after an elapse of a long period are listed in the health risk estimation table T after 10 years.

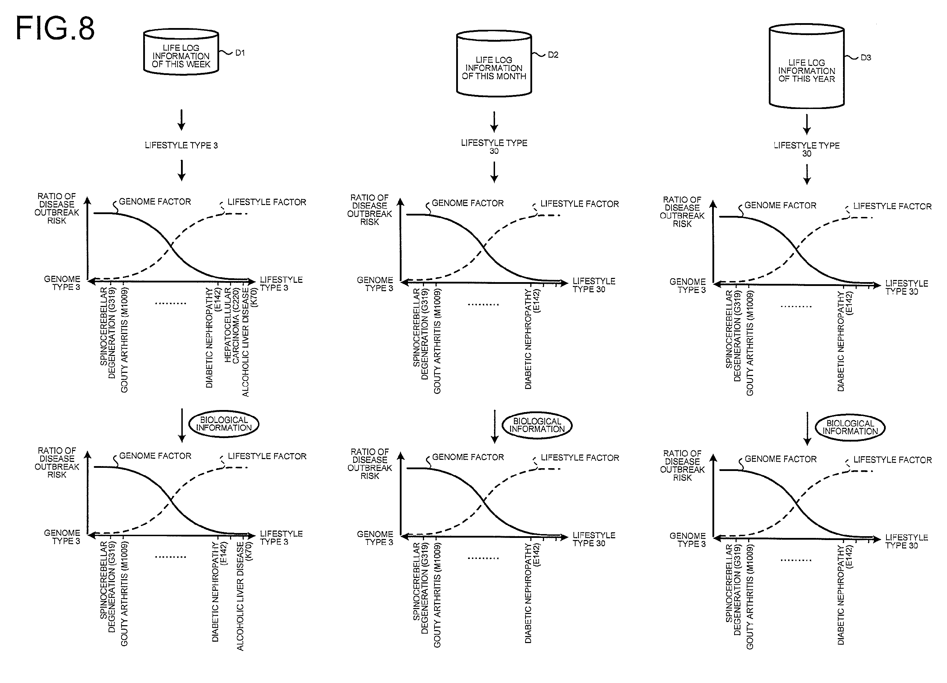

FIG. 8 is a diagram that illustrates the estimation of a health risk according to this embodiment. For example, when the health risk of user A is to be estimated, the primary use service providing unit 122 extracts life log information according to the estimation target period from the PHR data of user A. For example, the primary use service providing unit 122, as illustrated in FIG. 8, according to the estimation target periods designated by an operator, for example, extracts life log information D1 of this week, life log information D2 of this month, and life log information D3 of this year from the PHR data of user A.

Subsequently, the primary use service providing unit 122, for each estimation period, acquires values of ten items (smoking, drinking, sleep, stress, exercise, a food life, a medicine/supplement, a mental state, fatigue, and immunity) and derives the levels of the items based on these values. Then, the primary use service providing unit 122, for each estimation target period, determines the type of lifestyle that is one of a pattern of a combination of the level of each item as the type (the type of lifestyle of this week, the type of lifestyle of this month, and the type of lifestyle of this year) of lifestyle of user A. For example, the primary use service providing unit 122, as illustrated in FIG. 8, determines the type "type 3" of lifestyle of this week based on the life log information D1 of this week, determines the type "type 30" of lifestyle of this month based on the life log information D2 of this month, and determines the type "type 30" of lifestyle of this year based on the life log information D3 of this year.

Next, the primary use service providing unit 122 specifies a corresponding health risk graph for each estimation target period by referring to the health risk estimation table T using the determined type of lifestyle. For example, in the example illustrated in FIG. 8, in the health risk graph of lifestyle type 3, "alcoholic liver disease (K70)", "hepatocellular carcinoma (C220)", and "diabetic nephropathy (E142)" are listed as diseases having high outbreak risks. However, in the health risk graph of lifestyle type 30, "alcoholic liver disease (K70)" and "hepatocellular carcinoma (C220)" are excluded from the diseases having high outbreak risks, and only "diabetic nephropathy (E142)" is listed as a disease having a high outbreak risk. While this is merely an example for the convenience of description, in this way, in a case where the type of lifestyle is different for each estimation target period, the kinds of diseases having high outbreak risks and the order thereof are different for each estimation target period. By performing individual estimation according to each estimation target period and, for example, comparing the results of the estimations of this week, this month, and this year, the directivity (for example, in a good direction, a bad direction, or the like) of the health risk can be presented.

In addition, the primary use service providing unit 122 performs an adjustment of each health risk graph specified for each estimation target period according to the health state of the present. For example, the primary use service providing unit 122 changes the content of each health risk graph to a content according to the health state of the present of the individual user in consideration of the biological information included in the life log information. For example, it is assumed that the primary use service providing unit 122 analyzes the biological information of user A and determines that the liver function of user A is extremely satisfactory state. Then, the primary use service providing unit 122, in the health risk graph of a combination of the genome type 3 and the lifestyle type 3, determines that an outbreak risk of "hepatocellular carcinoma (C220)" among "alcoholic liver disease (K70)", "hepatocellular carcinoma (C220)", and "diabetic nephropathy (E142)" is low and removes "hepatocellular carcinoma (C220)". While this is merely an example for the convenience of description, in this way, the kinds of diseases having high outbreak risks and the order thereof are changed based on the health state of the present.

When the primary use service providing unit 122 according to this embodiment performs estimation of the health risk, individual estimation according to the estimation target period and the adjustment according to the health state of the present as described above are performed. In the example described above, as the estimation target periods, while "this week", "this month", and "this year" are used, the embodiment is not limited thereto. Thus, the estimation target period may be a period that is divided into predetermined units such as "yesterday", "past one week", "past one month", and "past one year". Alternatively, an appropriate setting may be received from a user, and an arbitrary period according to a user's desire may be used as the estimation target period.

Until now, the PHR big data analyzing unit 121 has been described to generate the "health risk estimation table T" representing the ratios of the genome factor and the lifestyle factor in the disease outbreak risk in accordance with a combination of the type of genome and the type of lifestyle. In addition to this, the PHR big data analyzing unit 121 may generate information representing a lifestyle that becomes a factor further increasing the disease outbreak risk for the "disease having a strong influence of the genome factor".

Until now, in a case where SNP is included in an ALDH2 genome, it is known that the outbreak risk of esophageal cancer is increased when there is a smoking habit and a drinking habit. From this, for example, by analyzing diseases having high outbreak risks for each lifestyle for a user of the type of genome having SNP in a specific genome, a correlation between a disease caused due to the inclusion of SNP and the lifestyle can be estimated.

In such a case, for example, the PHR big data analyzing unit 121 searches for a user having the type of genome including SNP in a specific genome based on the genome information. Then, the PHR big data analyzing unit 121 specifies diseases having high outbreak risks by referring to the clinical history information (for example, it can be acquired from the electronic medical record information or the like) of a user having the type of genome including SNP in a specific genome. Subsequently, the PHR big data analyzing unit 121 specifies lifestyles increasing the outbreak risk of a specific disease by referring to the life log information of the user having the type of genome including SNP in a specific genome.

In the embodiment described above, while the health risk graph has been described to be generated in consideration of "a person who is in the standard health state", the embodiment is not limited thereto. For example, it is known that there are complications such as nephropathy, retinopathy, and neuropathy in diabetes. In addition, it is known that there are complications such as a cerebral stroke, various heart diseases, and nephropathy in hypertension. Furthermore, it is known that there are complications such as bacterial pneumonia, influenza encephalopathy, and myocarditis in influenza. As above, in a case where there are complications in a disease, in a health risk graph of a person having such a disease, it is considered that the outbreak risk of such complications is increased. Thus, for example, the PHR big data analyzing unit 121 classifies persons who have diseases each having complications and performs a cohort analysis, whereby, for example, a health risk graph dedicated for a person considered as "a person having diabetes", "a person having hypertension", or "a person having influenza" can be generated. In addition, in such as case, the "daily medical checkup" service is provided for "a person having diabetes", "a person having hypertension", or "a person having influenza", the primary use service providing unit 122 can specify diseases having high outbreak risks by referring to the health risk graph that is dedicated for this patient.

### (Daily Medical Checkup - Future Health Risk Notification)

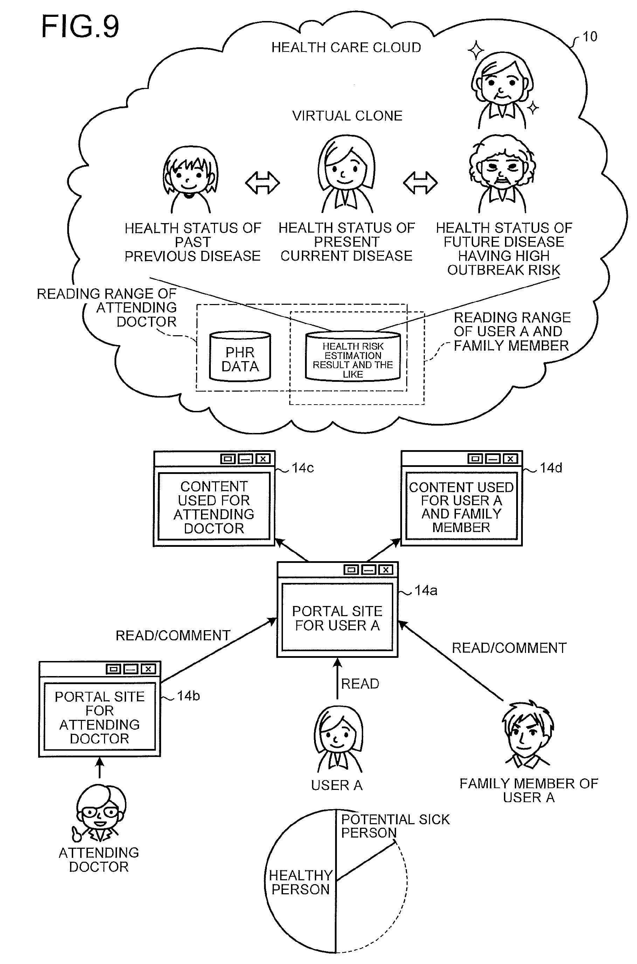

In this embodiment, the primary use service providing unit 122 performs feedback for the user providing the PHR data by using the health risk estimation table T, thereby providing the "daily medical checkup" as a primary use service. As a technique for the provision, while various techniques may be considered, hereinafter, one technique will be described with reference to FIG. 9.

FIG. 9 is a diagram that illustrates a health forecast portal site according to this embodiment. As illustrated in FIG. 9, for example, the primary use service providing unit 122 starts up a portal site 14a used for user A on the health care cloud 10 and permits user A and his family members to access the portal site 14a. In addition, for example, the primary use service providing unit 122 starts up a portal site 14b for an attending doctor on the health care cloud 10 and permits the attending doctor to access the portal site 14a used for user A through the portal site 14b for the attending doctor. In this way, by accepting accesses from user A, the family members, and the attending doctor through the portal site 14a used for user A, feedback to user A and information sharing among third parties are realized.

In addition, as illustrated in FIG. 9, in this embodiment, a range that can be read through the portal site 14a is different between the attending doctor and user A (and his family members). In other words, the attending doctor can read both the PHR data of user A and a result of the estimation of a health risk that is based on the PHR data. On the other hand, user A and his family members cannot read the PHR data of user A. For example, the reason for this is that the disclosure of the genome information of a person to the person needs to be appropriately restricted. The restriction of the range of reading is merely an example, and any other restriction may be applied. However, generally, it is considered that there are many cases where the reading range of the attending doctor is wider than the reading range of the person.

In addition, based on the opinion of the attending doctor, the reading range for user A and the family members may be adjusted. For example, the primary use service providing unit 122, among estimation results of the health risk, receives designation of items that are preferably read by user A and items that are not preferably read by user A from the attending doctor. Then, the primary use service providing unit 122, according to the designation made by the attending doctor, adjusts the reading range to be read by user A. For example, the primary use service providing unit 122 does not display some of diseases, which are displayed in a case where a health risk graph used for the attending doctor is displayed, in a case where a health risk graph used for the user is displayed. In this embodiment, there is a possibility that also a disease that is strongly influenced by the genome factor of the user is determined as a disease having a high outbreak risk. However, a situation may be considered in which the disease that is strongly influenced by the genome factor cannot be avoided by even changing the lifestyle, and, for example, in the case of an incurable disease for which a treatment has not been set, a notification thereof for the person does not mean anything (it may be adversely influenced). Thus, the primary use service providing unit 122, in a case where the health risk graph used for the user is to be displayed, does not display some of the diseases. For example, the primary use service providing unit 122 receives designation of diseases not to be displayed from the attending doctor, and such designation is reflected on the display of the health risk graph so as not to display the designated diseases. In addition, non-display of diseases is not limited to incurable diseases, but, for example, a case may be considered in which the attending doctor thinks that a notification for the person is not desirable in consideration of the characteristics of the person. Also in such a case, for example, the primary use service providing unit 122 receives designation of diseases not to be displayed from the attending doctor and reflects the designation on the display of the health risk graph so as not to display the designated diseases.

As above, the reading range is different between the attending doctor and user A and the family members, and, originally, the purpose of reading is different between the attending doctor and user A and the family members. Thus, in this embodiment, as illustrated in FIG. 9, a content 14c used for the attending doctor and a content 14d used for user A and the family members are separated prepared. This point will be described in detail when a screen transition is described below.

In addition, in this embodiment, the primary use service providing unit 122 presents the estimation result of the health risk as one or more of a "health risk graph", a "virtual clone", a "health status", a "mark that visually represents the health risk", and "character information".

For example, the primary use service providing unit 122 presents the estimation result of the health risk using the "virtual clone" associated with the PHR data of user A. For example, the "virtual clone" is set with being associated with each time point from the past to the future and maintains the health state at each time point in the form of a health status in which a point is given to each region. For example, the primary use service providing unit 122 appropriately extracts diseases having strong influences of the lifestyle factor from the health risk graph and calculates a point for each region by performing weighting according to the kind of each disease. In addition, in a case where a disease of a region has an influence on the other region, the primary use service providing unit 122 calculates points in consideration of such a point. For example, the "virtual clone" maintains an image of an expression according to the health status. In this way, visualization of distances from diseases is realized.

For example, the "virtual clone" of the past maintains the past health state and the health status according to the type of lifestyle, that are determined based on the past PHR data, and information of previous diseases. The "virtual clone" of the present maintains the health state of the present and the health status according to the type of lifestyle, that are determined based on the PHR data of the present, and information of current diseases. The "virtual clone" of the future maintains a future health status acquired by adding the type of lifestyle of the present to the health state of the present determined based on the PHR data of the present and information of diseases having high outbreak risks in the future. In addition, in this embodiment, an ideal "virtual clone" for user A is set and presented as well.

For example, user A or the attending doctor can acquire the health state of user A from the past to the future by accessing the portal site 14a used for user A and reading the "virtual clone" of user A. For example, user A or the attending doctor can acquire the clinical history of the user and the severity of an illness by moving the time of the "virtual clone" to the past. In addition, for example, user A or the attending doctor can display a future health risk that is premised on the lifestyle of the present of the user by moving the time of the "virtual clone" to the future.

In addition, for example, the primary use service providing unit 122 presents an estimation result of the health risk as a "mark that visually represents the health risk". This mark, for example, is a mark according to the health status and preferably is a mark that can be easily recognized by the user such as a "devil" of a case where the health status is bad and an "angel" of a case where the health status is good. In addition, for example, the primary use service providing unit 122 presents an estimation result of the health risk as "character information". For example, the primary use service providing unit 122 appropriately extracts diseases having strong influences of the lifestyle factor from the health risk graph and presents the extracted diseases to be aligned. Alternatively, in the "virtual clone" described above, by presenting a self-image acquired by reflecting a characteristic look predicted from the future health state on the face or the appearance of a person, a future image of the person after X years on which the present life is influenced may be displayed.

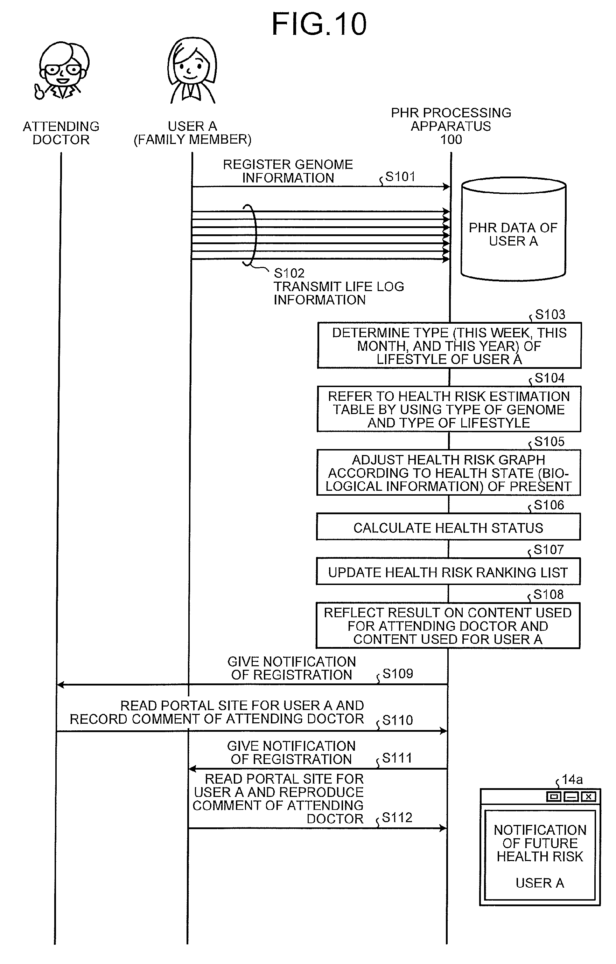

FIG. 10 is a diagram that illustrates the processing sequence of the "daily medical checkup" according to this embodiment. As illustrated in FIG. 10, it is assumed that user A registers the genome information in the PHR processing apparatus 100 in advance (Step S101). The process of this Step S101 is basically a process that may be performed at least once and is a process in which the process of Step S102 and subsequent steps are repeatedly performed.

In addition, as illustrated in FIG. 10, user A transmits the life log information collected from sensors and other information terminals from the wearing-type information terminal to the PHR processing apparatus 100 on a daily basis (Step S102). The PHR accumulation unit 110 of the PHR processing apparatus 100 accumulates the received life log information as the PHR data of user A on a daily basis and manages the accumulated life log information in a unified manner.

The primary use service providing unit 122, for example, performs the process of Step S103 and subsequent steps at the frequency of once per week. First, the primary use service providing unit 122 determines the type of lifestyle of user A for each estimation target period of the health risk (Step S103). For example, the primary use service providing unit 122 extracts the life log information D1 of this week, the life log information D2 of this month, and the life log information D3 of this year from the PHR data of user A and determines the type of lifestyle of user A for each estimation target period.

Subsequently, the primary use service providing unit 122 refers to the health risk estimation table T for each estimation target period of the health risk by using the type of genome of user A, which has been determined in advance, and the type of lifestyle determined in Step S103 (Step S104). For example, the primary use service providing unit 122 refers to the health risk estimation table T and, in a case where the type of genome of user A is type 3, and the type of lifestyle of this week is type 3, specifies the health risk graph illustrated in (B) of FIG. 7. In this way, the primary use service providing unit 122 specifies a health risk graph for each estimation target period.

Next, the primary use service providing unit 122 adjusts the health risk graph acquired in Step S104 in accordance with the health state of the present of user A for each estimation target period (Step S105). For example, in a case where the liver function of user A is determined to be in an extremely good state based on the biological information of user A, the primary use service providing unit 122 determines that the outbreak risk of "hepatocellular carcinoma (C220)" is low and removes "hepatocellular carcinoma (C220)" from the health risk graph of this week.

Then, the primary use service providing unit 122 calculates the health statuses of the present to the future for each estimation target period (Step S106) and registers the calculated health statuses in the "virtual clones" of the present to the future that are prepared for each estimation target period (Step S107). For example, the primary use service providing unit 122 calculates the health status of the present of user A based on the health status of the present calculated in the previous week and the biological information of this week and registers the calculated health status of the present in association with the "virtual clone" of the present of user A. In addition, the primary use service providing unit 122 calculates a health status of the future by combining a subtracted point accompanied with aging, a subtracted point accompanied with the health risk of the future determined in Step S105, and the like with the health status of the present being used as the reference and registers the calculated health status of the future in association with the "virtual clone" of the future of user A. In addition, when a health status of a future time point is calculated, the primary use service providing unit 122 calculates a health status of a middle time point between the present to the time point or a time point that is in the further future after the time point through appropriate interpolation (in a case where the health risk estimation tables T of a plurality of time points are prepared, by using the health risk estimation tables). For example, the primary use service providing unit 122 calculates a health status of each time point from after one day, after one week, or after one month to after one year, after five years, after 10 years, or after 20 years. In addition, the primary use service providing unit 122 calculates such a health status for each estimation target period.

In addition, the primary use service providing unit 122 updates a health risk ranking list maintained by the attending doctor of user A (Step S107). For example, the primary use service providing unit 122, for a plurality of users for whom the attending doctor is responsible, based on a health status after 10 years of a case where the estimation target period is "this year", generates a health risk ranking list in which the users are aligned in order of highest to lowest outbreak risk of a disease. Thus, the primary use service providing unit 122 updates this health risk ranking list based on the health status of "this year" that is calculated in Step S106.

Then, the primary use service providing unit 122 reflects the result of the process described above on the content used for the attending doctor and the content used for user A (step S108). For example, the primary use service providing unit 122 reflects the updated health risk ranking on the content used for the attending doctor. In addition, the primary use service providing unit 122 reflects the type of lifestyle of each estimation target period, the health risk graph of each estimation target period, and the health status of each estimation target period on the content used for user A.

Then, the primary use service providing unit 122 notifies the attending doctor of the registration (Step S109). The attending doctor, first, reads the health risk ranking at the portal site for the attending doctor. Then, for example, in a case where user A is positioned at high ranking in the health risk ranking, the attending doctor further reads the portal site for user A, records his comment, and uploads the recorded comment to the portal site for user A (Step S110). Here, the comment is not limited to moving image data but may be a comment using text data or the like.

Subsequently, the primary use service providing unit 122 notifies user A of the registration (Step S111), and user A reads the portal site for user A (Step S112). In a case where the comment of the attending doctor has already been recorded in Step S110, user A may reproduce the moving image as the comment of the attending doctor.

The processing sequence illustrated in FIG. 10 is merely an example. For example, in FIG. 10, while the processing sequence has been illustrated in which the process waits for a comment from the attending doctor, and user A is enabled to read the comment, the embodiment is not limited thereto. For example, the primary use service providing unit 122 may simultaneously notify three parties of the user, the family member, and the attending doctor of the registration of the portal site. In addition, the processing sequence illustrated in FIG. 10 may be performed not on the premise of the intervention of the attending doctor. Other than that, the setting of the estimation target period, the calculation of the health status, and the like may be arbitrarily changed in accordance with the provision form of services or may be omitted.

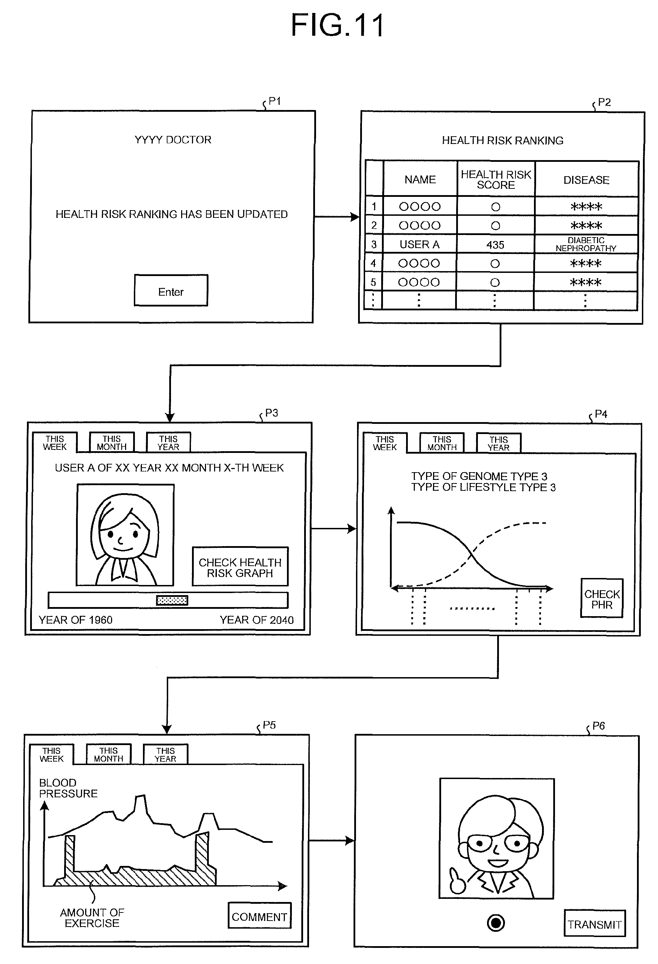

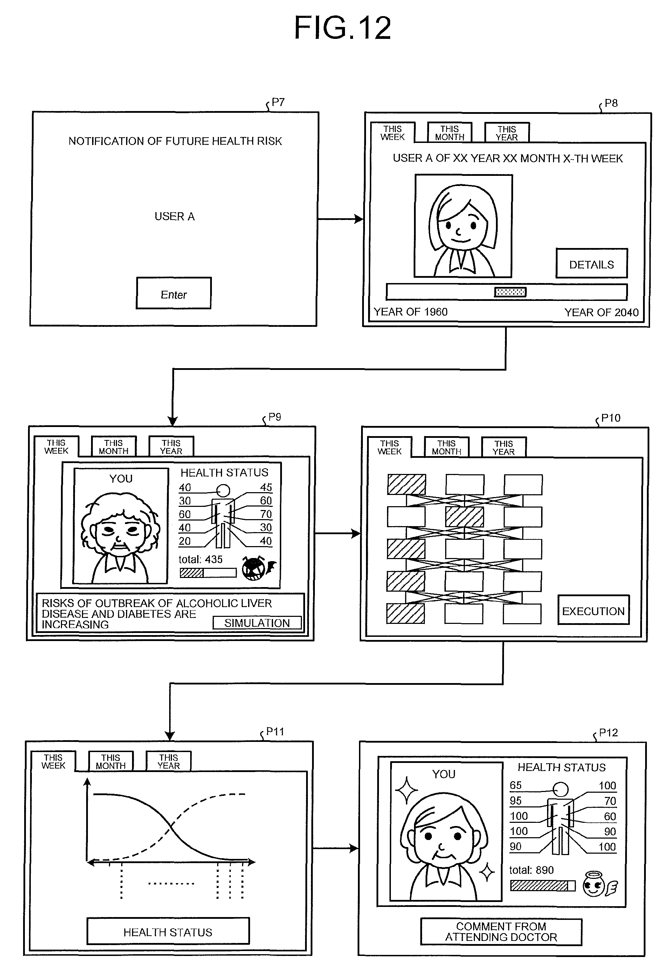

Next, at the portal site for the attending doctor or the portal site for user A, examples of contents to be read will be described according to screen transitions. FIG. 11 is a diagram that illustrates screen transitions at the portal site for the attending doctor according to this embodiment, and FIG. 12 is a diagram that illustrates screen transitions at the portal site for the user according to this embodiment. Here, the screen transitions illustrated in FIGS. 11 and 12 are merely examples, and the sequence of the screen transitions, the configuration of screens, and the like may be arbitrarily changed.

Here, the screen transitions illustrated hereinafter as examples are made in the PHR display apparatus 200 of the attending doctor or the PHR display apparatus 200 of user A. This is realized by a control process performed by the primary use service providing unit 122 and, at the same time, is realized by a display control process performed by the display control unit 210 arranged on the PHR display apparatus 200 side.

First, the screen transitions on the attending doctor side will be described. The attending doctor accesses the portal site for the attending doctor by using the PHR display apparatus 200. Then, as illustrated in screen P1 illustrated in FIG. 11, an update of the health risk ranking list is notified. Thus, the attending doctor presses a button 'Enter' and reads the health risk ranking list.

Then, as illustrated in screen P2, the primary use service providing unit 122 displays the health risk ranking on the PHR display apparatus 200 of the attending doctor. In the health risk ranking, user names, health risk scores, and the names of diseases having high outbreak risks are displayed in order of lowest to highest health risk score. For example, it is assumed that the name of user A is included in a high rank of the health risk ranking.

In such a case, the attending doctor selects the name of user A on the health risk ranking and accesses the portal site for user A. Then, as illustrated in screen P3, the primary use service providing unit 122 displays the portal site for user A on the PHR display apparatus 200 of the attending doctor. For example, the primary use service providing unit 122 displays a "virtual clone" of the present of user A. In addition, as illustrated in screen P3, tabs (tabs of "this week", "this month", and "this year") used for selecting an estimation target period are set on the screen. Here, in the description, it is assumed that the attending doctor selects "this week" as the estimation target period. In addition, under the "virtual clone", a bar is displayed as a tool used for receiving a time point desired to be checked. For example, the attending doctor adjusts the position of this bar to "Year 2023" that is after 10 years and presses a button of 'check the health risk graph'.

Then, as illustrated in screen P4, the primary use service providing unit 122 displays, together with the type of genome of user A and the type of lifestyle of this week, a corresponding health risk graph on the PHR display apparatus 200 of the attending doctor. In addition, while not illustrated in the figure, the primary use service providing unit 122 may specifically display the content of each item of the type of lifestyle as is necessary. Then, for example, after checking the health risk graph, the attending doctor presses a button of 'PHR checking'.

Then, as illustrated in screen P5, the primary use service providing unit 122 displays the PHR data of user A. In addition, in screen P5, while an example is illustrated in which the life log information is displayed in a graph form, the embodiment is not limited thereto. The primary use service providing unit 122 can process the PHR data designated by the attending doctor in a form (for example, a table form) desired by the attending doctor and displays the processed PHR data. For example, when the health risk graph of each estimation target period and the PHR data are schematically checked, the attending doctor presses a button of 'comment'.

Then, as illustrated in screen P6, the attending doctor records a comment moving image, for example, by using a recording function of the PHR display apparatus 200 and, by pressing a button of 'transmission', uploads the comment moving image.

The screen transitions described above will be described as below from a viewpoint of display control performed by the display control unit 210 of the PHR display apparatus 200. For example, the PHR display apparatus 200 of the attending doctor includes the display control unit 210 that displays the user's future health risk estimated based on the user's PHR data on the display unit 220. The display control unit 210 displays the health risk ranking list based on a comparison among a plurality of users and, in a case where a predetermined user is designated from the health risk ranking list, displays the future health risk and the PHR data of the designated user. The future health risk, for example, is displayed as a virtual clone, a health risk graph, any other character information, or the like. In addition, the PHR data is displayed as a graph form, a table form, any other character information, or the like. In addition, the display control unit 210 displays the type of genome and the type of lifestyle as the PHR data of the user. In addition, while not illustrated in FIG. 11, in a case where the name of a disease is to be displayed, the display control unit 210 displays the name as a formal name or an ICD code.

Next, the screen transitions on user A side will be described. User A accesses the portal site for user A by using the PHR display apparatus 200. Then, a screen represented as screen P7 illustrated in FIG. 12 is displayed, and thus, user A starts reading by pressing the button of 'Enter'.

Then, as illustrated on screen P8, the primary use service providing unit 122 displays the "virtual clone" of the present of user A. In addition, as illustrated in screen P8, tabs (tabs of "this week", "this month", and "this year") used for selecting an estimation target period are set on the screen. Here, in the description, it is assumed that user A selects "this week" as the estimation target period. In addition, under the "virtual clone", a bar is displayed as a tool used for receiving a time point desired to be checked. For example, user A adjusts the position of this bar to "Year 2023" that is after 10 years and presses a button of 'details'.

Then, as illustrated in screen P9, the primary use service providing unit 122 displays a "virtual clone" of the time point designated by user A and the health status of the time point. In addition, the primary use service providing unit 122, as an estimation result of the health risk, displays that "After 10 years (the year of 2023), the outbreak risks of "alcoholic liver disease" and "diabetes" are increased". In addition, the primary use service providing unit 122 displays a mark that visually represents the health risk. In the example illustrated in screen P9, as a meaning visually representing that the outbreak risk of a severe disease is increased, a mark of "devil" is displayed. Here, for example, the user presses a button of 'simulation'.

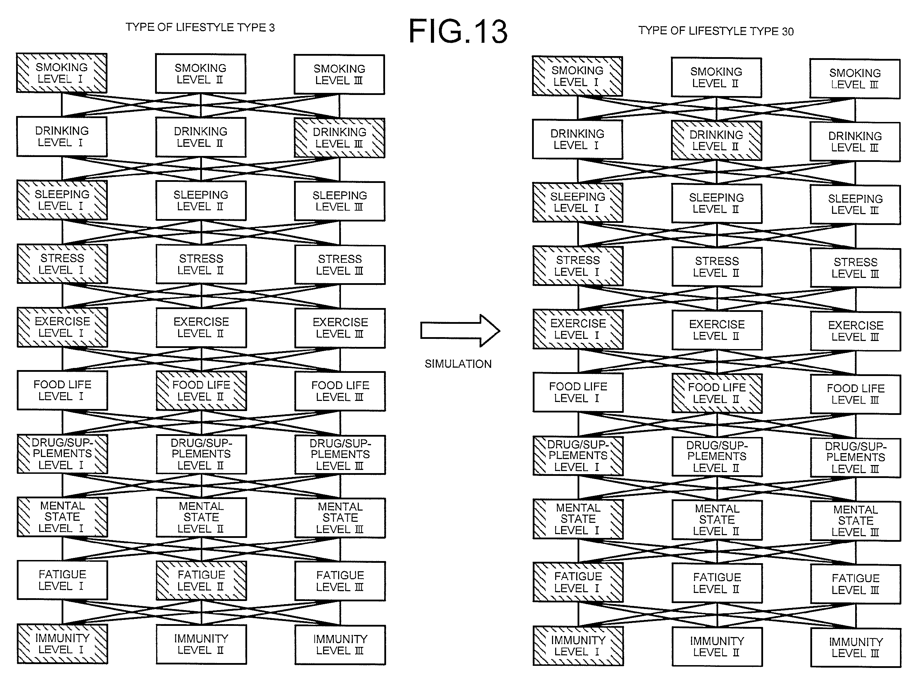

Then, as illustrated in screen P10, the primary use service providing unit 122 receives a change in the lifestyle and displays a simulation screen simulating the health risk. FIG. 13 is a diagram that illustrates a simulation of the health risk according to this embodiment. For example, the primary use service providing unit 122, as illustrated in FIG. 13, displays a GUI (Graphical User Interface) from which three steps of "level I" to "level III" can be selected for 10 items acquired from the life log information. In the GUI illustrated in FIG. 13, each level of each item is configured as a button that can be selected by being pressed by the user. While the primary use service providing unit 122, first, as illustrated on the left side in FIG. 13, displays the type of the lifestyle of the present of user A in a selected state, as illustrated on the right side in FIG. 13, the primary use service providing unit 122 receives a pressing operation from user A and changes the type of lifestyle. Here, an example is illustrated in which, for example, user A decreases the level of the item "drinking" from "level III" to "level II" and decreases the level of the item "fatigue" from "level II" to level "I". In addition, as a result of the selection made by user A, it is also represented that the type of lifestyle is changed to type 30. Here, the GUI used for the simulation is not limited to the example illustrated in FIG. 13. For example, the GUI may be changed to a pull-down menu or the like.

In this way, when the type of lifestyle desired to be simulated is selected, user A presses a button of 'execution' on screen P10 illustrated in FIG. 12. Then, the primary use service providing unit 122, together with specifying a health risk graph corresponding to the type of lifestyle that is simulated, adjusts the health risk graph according to the health state of the present of user A and, as illustrated in screen P11, displays the health risk graph after the simulation.

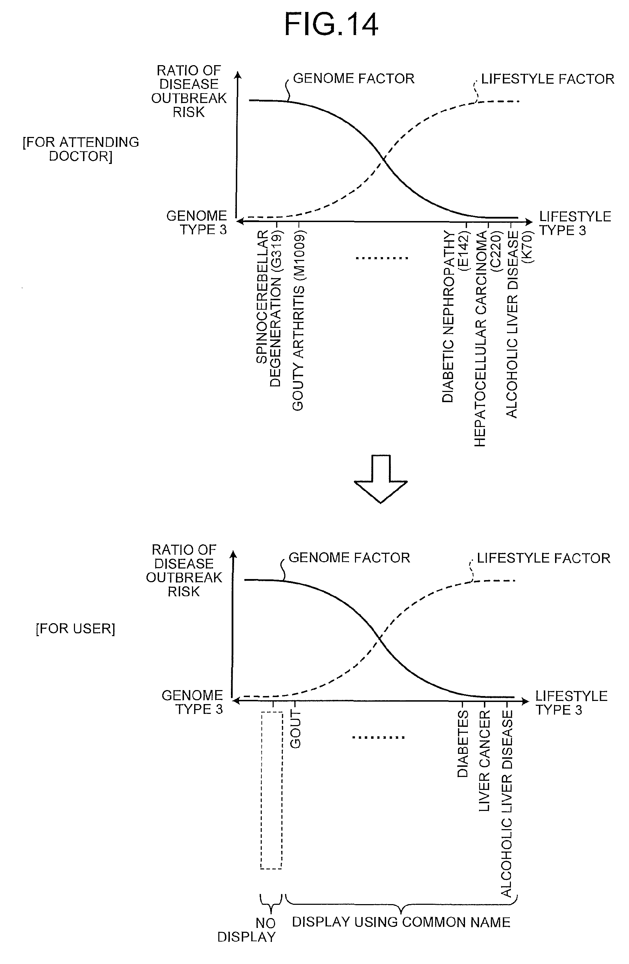

Here, the primary use service providing unit 122 changes the display form between a case where the health risk graph is displayed for a doctor such as the attending doctor and a case where the health risk graph is displayed for the user. FIG. 14 is a diagram that illustrates health risk graphs displayed for the attending doctor and the user in this embodiment. Here, points for changing the display form are mainly the following two points.

First, the first point is the display form of names of diseases. As illustrated in FIG. 14, in a case where the health risk graph used for the attending doctor is to be displayed, the primary use service providing unit 122 displays a formal name and an ICD code of a disease. On the other hand, in a case where the health risk graph for the user is to be displayed, the primary use service providing unit 122 displays a common name of a disease. For example, the primary use service providing unit 122 displays a disease displayed as "hepatocellular carcinoma (C220)" in the health risk graph used for the attending doctor as "liver cancer" in the health risk graph used for the user. In addition, for example, the primary use service providing unit 122 displays a disease displayed as "diabetic nephropathy (E142)" in the health risk graph used for the attending doctor simply as "diabetes" in the health risk graph used for the user. The primary use service providing unit 122 maintains correspondence between the formal names and the ICD codes and the common names in advance and appropriately performs replacement in displaying the health risk graph by referring to the correspondence.

Next, the second point is non-display of diseases. As illustrated in FIG. 14, the primary use service providing unit 122 sets some of diseases displayed in a case where the health risk graph used for the attending doctor is displayed not to be displayed in a case where the health risk graph used for the user is displayed. In other words, as described above, in this embodiment, there is a possibility that also a disease strongly influenced by the genome factor of the user is determined as a disease having a high outbreak risk. However, a situation may be considered in which the disease that is strongly influenced by the genome factor cannot be avoided by even changing the lifestyle, and, for example, in the case of an incurable disease for which a treatment has not been set, a notification thereof for the person does not mean anything (it may be adversely influenced). Thus, the primary use service providing unit 122, in a case where the health risk graph used for the user is to be displayed, may not display some of the diseases. For example, the primary use service providing unit 122 sets a disease "spinocerebellar degeneration (G319)", which is displayed in a case where the health risk graph used for the attending doctor is displayed, not to be displayed in a case where the health risk graph used for the user is displayed. In addition, for example, the primary use service providing unit 122 maintains a list of incurable diseases having strong influences of the genome factor in advance and sets the diseases not to be appropriately displayed in displaying the health risk graph by referring to this list. Alternatively, for example, the primary use service providing unit 122 receives designation of diseases not to be displayed from the attending doctor and reflects the designation on the display of the health risk graph so as not to display the designated diseases.

For example, when the health risk graph after the simulation is checked, user A presses a button of 'health status'. Then, as illustrated in screen P12, the primary use service providing unit 122 displays the "virtual clone" and the health status after the simulation. For example, by executing the content of the simulation by checking the expression of the "virtual clone" after the simulation and the health status after the simulation, user A can recognize that the health risk and the health status are improved. For example, user A can recognize that the outbreak of the "alcoholic liver disease" and the "liver cancer" can be avoided by switching the life to a life slightly refraining from drinking and taking a sufficient rest. In addition, the primary use service providing unit 122 displays a mark of "angel" as a mark visually representing that an outbreak risk of a serious disease decreases. Furthermore, for example, in a case where a comment is uploaded from an attending doctor, the primary use service providing unit 122 displays a button of 'comment from attending doctor' on screen P12. Then, user A can check the comment of the attending doctor by pressing the button of 'comment from attending doctor'.

The screen transitions described above will be described as below from a viewpoint of display control performed by the display control unit 210 of the PHR display apparatus 200. For example, the PHR display apparatus 200 of user A includes the display control unit 210 that displays the user's future health risk estimated based on the user's PHR data on the display unit 220. The display control unit 210, together with the future health risk, displays at least one of the target health state of user A and guidance information for arriving at the target health state. The future health risk, for example, is displayed as a "virtual clone", a health status, a health risk graph, any other character information, or the like. In addition, the target health state is displayed as an ideal "virtual clone", an ideal health status, a health risk graph after the simulation, any other character information, or the like. Furthermore, the guidance information is displayed using a comment from the attending doctor, character information prepared in advance, or the like.

In addition, when designation of an estimation time point is received from the operator, the display control unit 210 displays a health risk of the future corresponding to the received time point. Furthermore, when the width of a period of the PHR data of the user that is used for the estimation is received from the operator, the display control unit 210 displays a health risk of the future according to the received width of the period. The health risk of the future according to the received width of the period may be prepared in advance for each period or may be prepared after the designation is received from the user. In addition, when an instruction for changing the lifestyle is received from the operator, the display control unit 210 further displays a health risk of the future that is simulated according to the received change instruction. Furthermore, the display control unit 210 displays the names of diseases that may be caused for user A in the future by using common names as the health risk of the future. In addition, in a case where the names of the diseases are displayed for user A or a family member of user A, the display control unit 210 does not display the names of some of the diseases as is necessary.

In the example illustrated in FIG. 12, while an example has been described in which user A performs a simulation changing the lifestyle and checks the health risk graph and the health status after the simulation, the embodiment is not limited thereto. For example, the primary use service providing unit 122 may present the ideal "virtual clone" together with a lifestyle proposed to user A and automatically propose enhancement of the lifestyle to user A.

As above, according to the "future health risk notification", a family member or the attending doctor can monitor the health of the body and the mind of the user through the "virtual clone". Then, an appropriate encouragement or guidance toward the ideal can be performed. The user can check a technique for being healthy and the progress status more specifically, and accordingly, the motivation can be increased further. In the embodiment described above, while a conversation or a response with/from the "virtual clone" is not considered, for example, by using a simulation technology altogether, a conversation or a response with/from the "virtual clone" can be realized. In such a case, not only a "virtual clone" of the user but also a "virtual clone" of a family member or a "virtual clone" of the attending doctor may be set. In such a "virtual clone", the conversation or the content of the guidance that is considered is set in advance. Then, also in a situation in which a comment is not actually given from a family member or the attending doctor, the user can acquire the comment. In addition, the user may have a conversation with his "virtual clone".

As described above, according to this embodiment, the future health risk according to the lifestyle of each individual and the continuation of the lifestyle can be presented with high accuracy by using the PHR data including the genome information. In addition, according to this embodiment, by estimating a food, an exercise, and a change in the lifestyle that are optimal and medicine and supplements that are effective for the individual, an environment for approaching a more healthy and ideal individuality can be brought. Furthermore, the checking of the degree of approach to the ideal individuality materializes the result of efforts, of which the target is not visible, and turns the result into a will and a joy. In addition, this embodiment can respond to disaster resilience for remotely checking and managing the presence, the existence status, and the condition when the condition deteriorates at a refuge site due to isolation according to a disaster or the like.

### (Other Use of "Health Risk Estimation Table T")

In the embodiment described above, as a specific example, an example has been described in which information of diseases having strong influences of the lifestyle factor is fed back as a result of the estimation of the health risk by referring to the "health risk estimation table T" by using the type of genome and the type of lifestyle of the user. However, the form of the use of information acquired from the "health risk estimation table T" is not limited thereto.

For example, based on the determination of diseases having high outbreak risks for a user, for example, items of the life log information collected from the user may be narrowed into items relating to the diseases having high outbreak risks and be intensively collected. For example, the types of sensors and the items used on the user side may be changed according to the type of the genome and the type of lifestyle of the user.

In addition, by referring to the "health risk estimation table T" by using the type of genome and the type of lifestyle of the user, information of diseases having strong influences of the genome factor, in other words, diseases that are genetically at a high risk according to the user can be estimated.

Furthermore, for example, the primary use service providing unit 122 may provide a mechanism for quickly supplementing indications of diseases having high outbreak risks for the user from the PHR data that is transmitted from the user on a daily basis. For example, the primary use service providing unit 122 arranges a threshold matching a specific disease and sequentially checks the PHR data transmitted from the user using the threshold.

For example, for a user for whom high-risk diseases are determined, for example, by concentrating the supplement of indications of severe diseases such as brain or heart diseases using all the sensors, there is a ripple effect bringing such a new preventive/preemptive medical revolution that an individual is warned and is urged to take a rest by acquiring signs, an attack is suppressed by preparing an early medical examination, and, even in the case of the occurrence of an attack, a speedy countermeasure or treatment can be performed in a low degree of the attack. Alternatively, users having a high outbreak risk for cardiac sudden death are selected using the genome information, the users are constantly monitored using the sensors, and an emergency system can be built for an instruction for a lifestyle for preventing an attack in advance, operating a pace maker only at the time of an occurrence of arrhythmia in an auxiliary manner, turning on an electronic pill in which an antiarrhythmic agent or an antithrombotic agent is filled, or instantly contacting a nearby medical institution at the time of deterioration of the condition for an emergency call, and an instruction for a place at which a nearby AED (Automated External Defibrillator) is present or support for relief at the time of a cardiac arrest, or the like. As a result, sudden death, an aftereffect lasting over a long period accompanied with seriousness after cardiac attack, or a bedridden life or dementia according to rehabilitation or a secondary damage can be prevented or reduced, and accordingly, also in an aging society, everyone can deal with hobbies, work, and housework without any concern for the health and spend a pleasant and peaceful life.

In addition, for a disease outbreak reserve of a genetically high risk such as signs of an outbreak of cerebral infarction accompanied with arrhythmia due to stress or the like, daily data monitoring is strengthened, and an abnormal log is caught without missing them, whereby early alarm information or a disease outbreak preventive measure can be fed back to each individual or a medical institution with high accuracy. In addition, information of a separated family member registered in advance can be fed back in this way. As a result, through a health guidance method for individualization that is based on an optimal evidence for each individual, a more appropriate selection of foods and exercises, and even a selection of a living mode, the ideal individuality can be realized more specifically, reasonably, and pleasantly, and watching for a family member is constantly performed as if he is present nearby, whereby a peaceful life can be realized.

### (Secondary Use Service)

Until now, as an example of the primary use service of the PHR data, while the "daily medical checkup" and the "future health risk notification" have been described, as described above, in this embodiment, the PHR processing apparatus 100 is considered to provide a secondary use service of the PHR data as well. For example, the PHR processing apparatus 100 acquires an analysis result representing predetermined relevance by analyzing the large-scale genome cohort database 114a such that the relevance between a combination of a type of genome and a type of lifestyle and a specific object can be derived and provides the analysis result for medical institutions, various companies, and the like.

In addition, the PHR big data accumulated in the large-scale genome cohort database 114a is PHR data that is originally collected from each individual, in other words, personal information. For this reason, regarding the use of the PHR data, there are cases where the intention of each individual is different such as "a primary use of the PHR data is permitted, but a secondary use thereof is not permitted" and "none of a primary use and a secondary use of the PHR data is permitted". Thus, in this embodiment, the PHR processing apparatus 100 accepts use permission representing the permitted range of uses from each individual providing the PHR data in advance and manages information of the use permission in accompaniment with the PHR data. The use permission may be accepted for the whole PHR data or for the PHR data in units of subdivided items of the PHR data. Hereinafter, on the premise that such use permission is acquired, specific examples of the secondary use service will be described. However, the specific examples described below are merely examples, and the secondary use service is not limited to the specific examples described below.

First, as a first example, an example will be described in which relevance between a combination of a type of genome and a type of lifestyle and a "drug effect" is derived, and the relevance is used for a prescription of a drug.

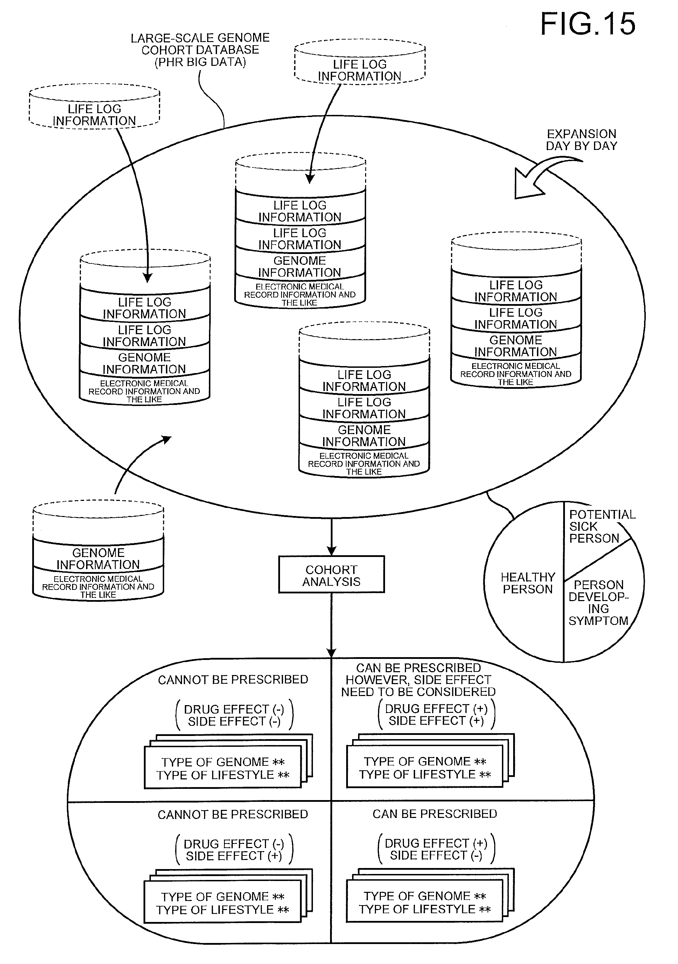

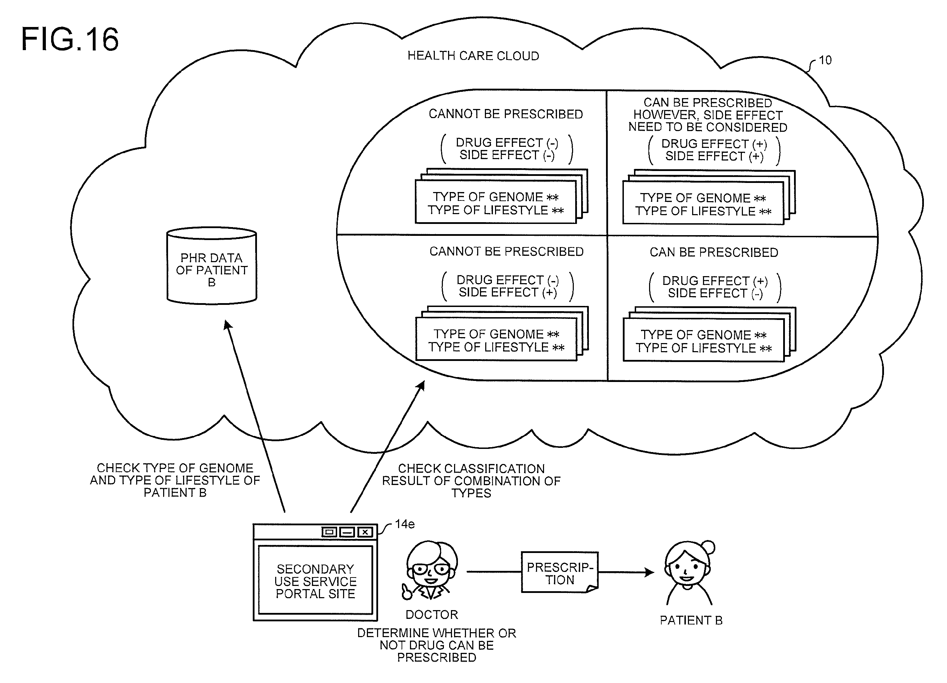

FIGS. 15 and 16 are diagrams that illustrate an example (first example) of the secondary use service according to this embodiment. As described above, in the large-scale genome cohort database 114a, the life log information that is the PHR data of each individual and the like are newly accumulated day by day, and the PHR data of a new individual is accumulated as a new operating/managing target, whereby the scale of the large-scale genome cohort database 114a increases day by day.

The PHR big data analyzing unit 121 receives an input of an object of the analysis that is a drug effect of a specific drug and performs a cohort analysis of the PHR big data accumulated in the large-scale genome cohort database 114a as the target, thereby deriving relevance between a combination of a type of genome and a type of lifestyle and a drug effect of a specific drug. For example, in the PHR data, the electronic medical record information is included, and, in the electronic medical record information, information of a drug prescribed to the individual and information representing the progress made thereafter are included. In addition, in the PHR data, the life log information is included, and, in the life log information, information representing a condition change of the individual after the prescription of the drug and information representing the life status of the individual are included.

Thus, the PHR big data analyzing unit 121 performs a cohort analysis of the PHR big data including such information as the target, thereby deriving relevance between a combination of a type of genome and a type of lifestyle and a drug effect and a side effect. Then, the PHR big data analyzing unit 121, as illustrated in FIG. 15, classifies combinations of a type of genome and a type of lifestyle based on the presence/no-presence of the drug effect and the side effect.

For example, the PHR big data analyzing unit 121 traces a group (a group matching a combination of a specific type of genome and a specific type of lifestyle) that is exposed to a specific factor and a group (a group not matching the combination) that is not exposed to the specific factor for a predetermined period and compares the presence/no-presence of the drug effect and the side effect, thereby deriving relevance between the factor (the combination of the specific type of genome and the specific type of lifestyle) and the drug effect and the side effect. Then, the PHR big data analyzing unit 121, based on the derived relevance, classifies combinations of a type of genome and a type of lifestyle into groups of "drug effect (-) and side effect (+)", "drug effect (-) and side effect (-)", "drug effect (+) and side effect (+)", and "drug effect (+) and side effect (-)".

Here, to a person having a type of combination classified into the groups of "drug effect (-) and side effect (+)" and "drug effect (-) and side effect (-)", the drug cannot be prescribed. In addition, to a person having a type of combination classified into the group of "drug effect (+) and side effect (+)", the drug can be prescribed, but the side effect need to be considered. In addition, to a person having a type of combination classified into the group of "drug effect (+) and side effect (-)", the drug can be prescribed. By providing such information for a doctor, the doctor can perform a determination according to the type of combination of a patient before prescribing the drug to the patient.

Thus, for example, the secondary use service providing unit 123, based on a contract exchanged with a medical institution in advance, provides a secondary use service for utilizing the relevance between a combination a type of genome and a type of lifestyle and the drug effect and the side effect for doctors of the medical institution. As a technique used for the provision, while various techniques may be considered, hereinafter, one technique will be described with reference to FIG. 16.

As illustrated in FIG. 16, for example, the secondary use service providing unit 123 starts up a portal site 14e for a secondary use service on the health care cloud 10 and permits a doctor of the medical institution to access the portal site 14e. In addition, the secondary use service providing unit 123 permits the doctor to access the PHR data of each individual and permits the doctor to access a classification result of combinations of a type of genome and a type of lifestyle. Then, the doctor, for example, reads the PHR data of patient B through the portal site 14e and checks the combination of the type of genome and the type of lifestyle of patient B. In addition, the doctor checks the classification result through the portal site 14e. Then, the doctor combines the classification result of the type of genome and the type of lifestyle of patient B and determines whether or not a specific drug is to be prescribed to patient B or whether or not it is necessary to consider the side effect in the prescription. Then, the doctor generates a prescription for patient B based on the determination.

However, the technique used for the providing the secondary use service is not limited to the technique described above. The secondary use service providing unit 123, for example, may be configured to generate a real name list including information of the type of genome and the type of lifestyle of each individual and a classification result for a specific drug and transmit the real name list and the classification result to the medical institution or the like. Here, the real name list and the classification result may be provided via offline.

Subsequently, as a second example, an example will be described in which relevance between a combination of a type of genome and a type of lifestyle and "effects of foods and supplements" is derived, and the derive relevance is utilized for the sales, advertisement, and the like of the foods and supplements.

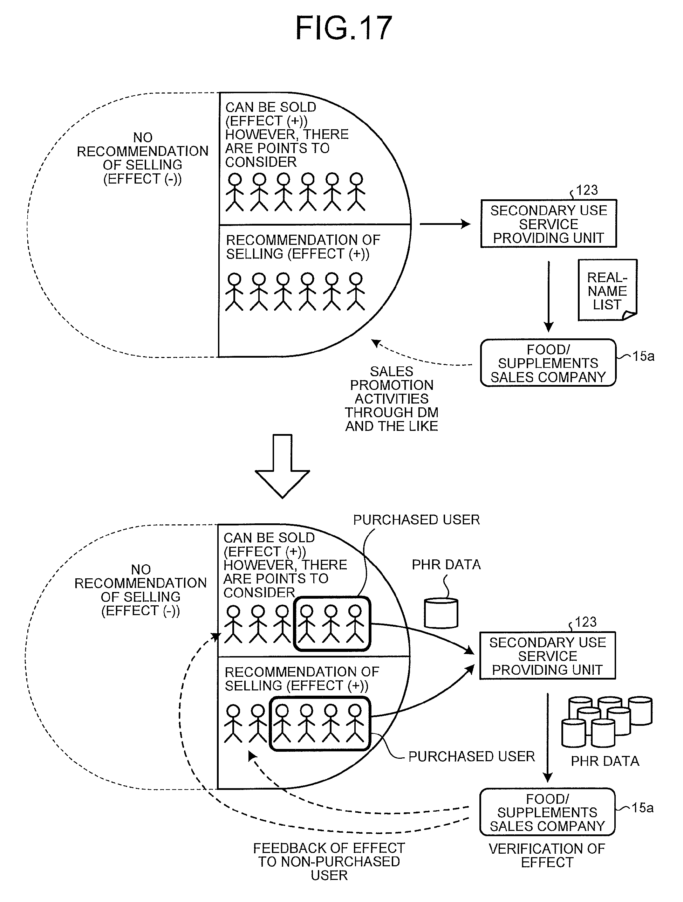

FIG. 17 is a diagram that illustrates an example (second example) of the secondary use service according to this embodiment. The PHR big data analyzing unit 121 receives an input of an object of the analysis that is an object of deriving relevance with an effect of a component (or a similar component thereof) contained in a specific health beverage and performs a cohort analysis of the PHR big data accumulated in the large-scale genome cohort database 114a as the target, thereby deriving relevance between a combination of a type of genome and a type of lifestyle and the specific health beverage. For example, in the PHR data, the life log information is included, and, in the life log information, information representing the condition of the individual and information representing the intake status of foods and supplements are included.

Thus, the PHR big data analyzing unit 121 performs the cohort analysis of the PHR big data including such information as the target, thereby deriving relevance between a combination of a type of genome and a type of lifestyle and the specific health beverage. For example, the PHR big data analyzing unit 121 traces a group (a group matching a combination of a specific type of genome and a specific type of lifestyle) that is exposed to a specific factor and a group (a group not matching the combination) that is not exposed to the specific factor for a predetermined period and compares the presence/no-presence of the effects of components (or similar components thereof) contained in the specific health beverage that is the target for the analysis, thereby deriving relevance between the factor (the combination of the specific type of genome and the specific type of lifestyle) and the specific health beverage.

Then, the PHR big data analyzing unit 121, based on the derived relevance, classifies combinations of a type of genome and a type of lifestyle into a group having an effect for a component (or a similar component thereof) contained in the specific health beverage and a group not having an effect for the component. In addition, the PHR big data analyzing unit 121 classifies the group having the effect into two groups in view of a combination with the intaking food.

In other words, it is not recommended to sell a specific health beverage to persons each having a combination of types classified into the group not having the effect for the health beverage. In addition, while the health beverage can be sold to persons each having a combination of the types classified into a group to which attention needs to be paid regarding a combination with the intaking foods and supplements among the group having the effect for the specific health beverage, it is preferable to report points to consider regarding the combination with the intaking foods. In addition, it is recommended to sell the health beverage to persons each having a combination of the types classified into the remaining groups.

Thus, for example, the secondary use service providing unit 123, based on a contract exchanged with a food/supplements sales company in advance, provides a secondary use service for utilizing the relevance between a combination a type of genome and a type of lifestyle and the health beverage for a food/supplements sales company 15a. As a technique used for the provision, while various techniques may be considered, hereinafter, one technique will be described with reference to FIG. 17.

As illustrated in FIG. 17, for example, the secondary use service providing unit 123 extracts users having combinations of types classified into groups to which the specific health beverage can be sold or is recommended to be sold from among a user group that is a provider of the PHR data. Then, the secondary use service providing unit 123 generates a real name list including points to be considered for such users and transmits the real name list to the food/supplements sales company 15a. Here, the real name list may be provided to be read through a portal site or may be provided via offline. Then, as illustrated in FIG. 17, the food/supplements sales company 15a performs sales promotion activities through direct mails (DM) or the like by using the real name list. In addition, the food/supplements sales company 15a reports points to be considered as is necessary in the sale promotion activities.

Next, for example, the secondary use service providing unit 123 traces PHR data corresponding to the users each having a combination of the types classified into the groups to which the specific health beverage can be sold or is recommended to be sold for a predetermined period. Then, the secondary use service providing unit 123 calculates users who have purchased the specific health beverage by using the life log information and transmits the PHR data of the purchasing users to the food/supplements sales company. There, the PHR data of the purchasing users may be provided to be read through a portal site or be provided via offline.

Then, as illustrated in FIG. 17, the food/supplements sales company 15a verifies the effect of the health beverage by using the PHR data. For example, the food/supplements sales company 15a calculates quantitative numerical values representing the effects. Then, the food/supplements sales company 15a feeds back the effects that are based on the calculated numerical values to users who have not purchased the health beverage among the users each having a combination of the types classified into the groups to which the specific health beverage can be sold or is recommended to be sold.

In addition, in the example described above, while an example has been described in which the secondary use service providing unit 123 calculates users who have purchased the specific health beverage by using the PHR data and provides the PHR data of the users who have purchased the specific health beverage for the food/supplements sales company 15a, the embodiment is not limited thereto.

For example, the secondary use service providing unit 123 may calculate users who have purchased the specific health beverage, narrow down the users into users actually having an effect by analyzing the PHR data, and provide a real name list of the narrowed-down users to the food/supplements sales company 15a. For example, in a case where the food/supplements sales company 15a desires to perform sales activities by narrowing down users having a remarkable effect into targets, such a narrowing-down process is effective. In addition, for example, the secondary use service providing unit 123 narrows down the types of lifestyle of users for whom an actual effect is acquired and specifies lifestyles relating to the effect. Then, the secondary use service providing unit 123 may provide information of the specified lifestyles to the food/supplements sales company 15a. In such a case, additionally, the food/supplements sales company 15a may propose a lifestyle at the time of selling foods or supplements.

Subsequently, as a third example, an example will be described in which the PHR data transmitted from a user is utilized for a family watching service for a family member who is separated far or the like.

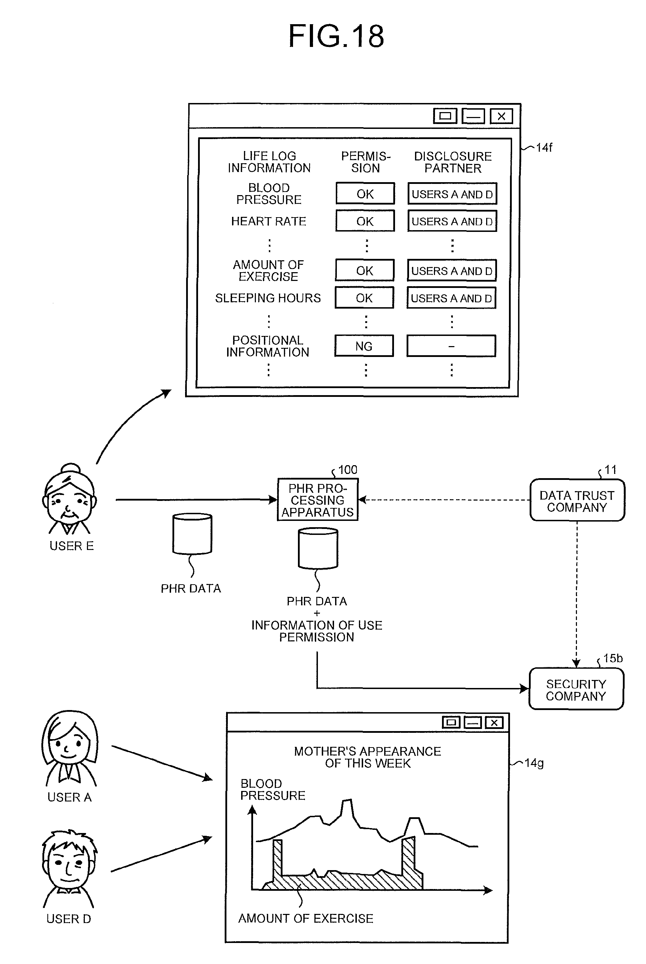

FIG. 18 is a diagram that illustrates an example (third example) of the secondary use service according to this embodiment. As illustrated in FIG. 18, for example, the secondary use service providing unit 123 receives use permission from each user who provides the PHR data in advance. The contents of this use permission, for example, are items of the life log information that are permitted to be disclosed (disclosure items) and a disclosure partner (disclosure destination).

For example, the secondary use service providing unit 123 receives use permission from user E who is an old person on a portal site 14f for user E. For example, in the example illustrated in FIG. 18, it is represented that the secondary use of the blood pressure and the heart rate among the life log information is permitted for the family watching service, and the disclosure partners of the information are user A who is a daughter of user E and user D who is a son of user E. Similarly, it is represented that the secondary use of the amount of exercise and the sleeping hours among the life log information is permitted for the family watching service, and the disclosure partners of the information are user A who is a daughter of user E and user D who is a son of user E. On the other hand, among the life log information, it is represented that the positional information is not permitted for the secondary use for the family watching service.

The secondary use service providing unit 123 transmits the information of such use permission received for the secondary use to the PHR accumulation unit 110. Then, the PHR accumulation unit 110 causes the information of the use permission in which the above-described contents are described to be in accompaniment with the PHR data transmitted from user E and stores the information. In addition, a method of causing the use permission information to be accompanied may use a technique of causing the use permission information to be accompanied with the whole PHR data or a technique of causing the use permission information to be accompanied with data of each subdivided item. Any arbitrary technique may be used for a method of causing the use permission information to be accompanied such that the use permission information can be checked on the side on which the PHR data is used.

In addition, the secondary use service providing unit 123 processes the PHR data provided as above into a form that is appropriate for the family watching service or the like and supplies the processed PHR data to a security company 15b that provides the family watching service. For example, the secondary use service providing unit 123, for blood pressure, a heart rate, an amount of exercise, and sleeping hours corresponding to one week, processes each value into a form such as a graph in which each value is plotted in a time series sequence such that a trend for one week can be easily checked and supplies the PHR data after the processing to the security company 15b. In addition, a technique for the provision may be an online technique or an offline technique. In addition, the secondary use service providing unit 123 may be configured to supply the PHR data to the security company 15b. In such a case, the above-described processing is performed by the security company 15b as is necessary.

For example, the security company 15b operates the family watching service. For example, a subscriber of the family watching service is user A who is a daughter of user E who is an old person. In the contract with the security company 15b, user A determines that user E who is an old person is a watching target, and user D who is a son of user E desires to use the service in addition to user A. Here, the subscriber of the family watching service and the user using the service provided by the PHR processing apparatus 100 side do not necessarily coincide with each other.

In this way, for example, the security company 15b starts up a watching portal site 14g for user E who is an old person at the site thereof. An access to the watching portal site 14g is permitted only to user A and user D. Then, user A and user D, on the watching portal site, for example, can check the health state, the appearance of exercises, the sleeping status, and the like of the mother thereof for this one week.

As described above, in the third example, the secondary use service providing unit 123, for the PHR data transmitted from the user, receives use permission relating to disclosure items and a disclosure destination. Then, the secondary use service providing unit 123 outputs the user's PHR data or the processing information of the PHR data in accordance with the received use permission. In this way, based on the life log information of a family member who is separated far, a service providing the status of the family member even in a case where the family member is separated far can be provided. In addition, at that time, a mechanism in which only designated information among the life log information can be disclosed for a specific partner can be realized. Furthermore, the information of the use permission is accompanied with each life log information, and a method having data to which a mark indicating that the data may be disclosed to a specific partner can be attached is formed.

As above, as the secondary use of the PHR big data, the first, second, and third examples have been described. As such a secondary use progresses, not only a support service for returning only an evaluation result of a health risk of the future to the provider of the PHR data but a new business model can be built in which various economical merits such as provision of distribution and product selling services, design of a lifestyle, a system of returning points that can be used as local currency, and the like are returned to individuals, a local government, and the society.

As described above, in this embodiment, as the number of participants or the scale increases, verification cycles and evidences are accumulated, and the performance is improved as a system having higher reliability and creditability. In addition, since the system is configured by various kinds of data, the degree of usefulness is high. By processing such a database and promoting the secondary use for the industry in various forms, basic data that is based on the life log big data and the genome information, which has been accumulated only and of which the value cannot be sufficiently found until now, is enabled to have a new and groundbreaking value, and accordingly, a new revolutionary industry using such data can be created.

### (Incentive for Collecting PHR Data)

Until now, as examples of the primary use service of the PHR data, the "daily medical checkup" and the "future health risk notification" have been described. In addition, the secondary use service of the PHR data has been described with reference to the specific examples. In any case, it is preferable that the PHR data satisfying the amount of data and kinds of data required for the primary use or the secondary use is continuously transmitted from each individual. Thus, in this embodiment, the PHR processing apparatus 100 further builds an incentive mechanism for each individual to continuously transmit the PHR data.

First one of such mechanisms is a link to the secondary use service. In a case where a permission is acquired from a corresponding user, the PHR data transmitted from each user is supplied also for the secondary use service. Thus, the PHR processing apparatus 100, for example, based on earnings acquired from the data trust company 11 relating to the secondary use service, builds a feedback mechanism for each user in a form such as a point system (points, mileage, dividend, or the like) or the like.

Second one of such mechanisms is competition among users. Each user competes with competition partners such as friends and family members for a win, and accordingly, a will toward healthiness can be increased. Thus, the PHR processing apparatus 100 builds a competition mechanism for a win, for example, by comparing the data amount and the number of kinds of the PHR data, biological information such as weight or blood pressure, or behavior information such as a distance taken for a walk or the number of steps taken per day between competition partners. The competition partner may be a virtual friend, a virtual lover, a virtual family member, or the like that is virtually set.

Third one of such mechanisms is a health forecast described in the above-described embodiment. In other words, by supplying the PHR data to the PHR processing apparatus 100, each user can receive his future health risk that is estimated based on the PHR data as a health forecast. As described above, the health forecast presents a future health risk in various forms. For example, the user may estimate the health risk corresponding to an estimation target period such as this week, this month, this year, past one day, past one week, past one month, or past one year or may estimate the health risk by setting an arbitrary time point such as after one day, after one week, after one month, after one year, after five years, after 10 years, or after 20 years as a future time point.

Then, hereinafter, the first one and the second one of the mechanisms described above will be described in detail.

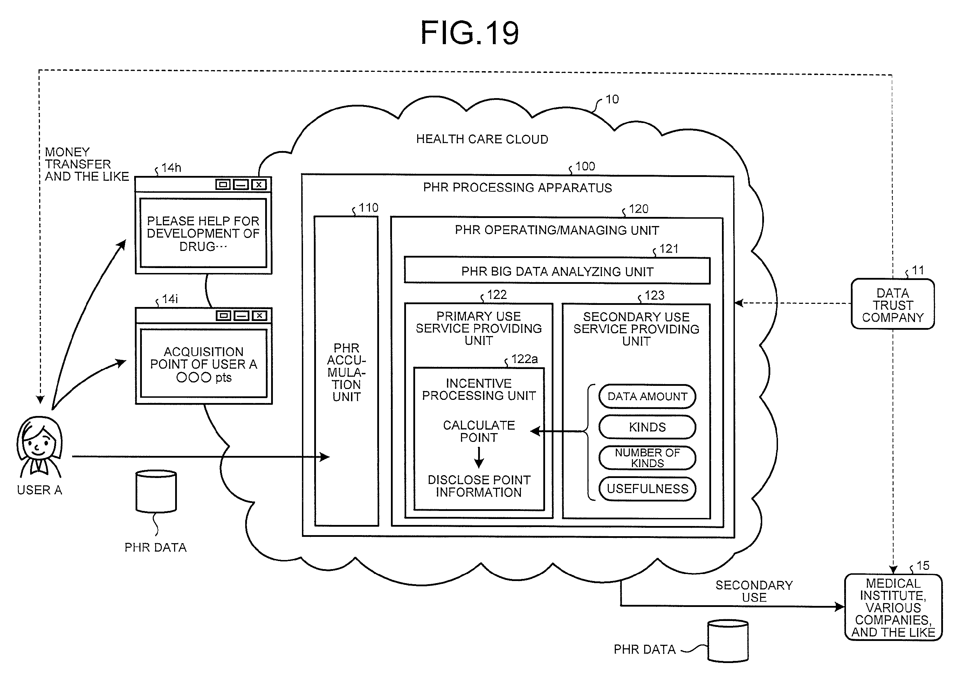

FIG. 19 is a diagram that illustrates a first one of incentive mechanisms according to this embodiment. As illustrated in FIG. 19, the primary use service providing unit 122 includes an incentive processing unit 122a (also referred to as a "presentation unit"). The incentive processing unit 122a evaluates the PHR data of a specific user and presents a result of the evaluation to the specific user. For example, the incentive processing unit 122a, in cooperation with the secondary use service providing unit 123, regarding the specific user who has permitted the secondary use of the PHR data, receives use information such as the amount of data, kinds, the number of kinds of the PHR data used for the secondary use and the usefulness of the secondary use service in which the PHR data is utilized from the secondary use service providing unit 123. Then, the incentive processing unit 122a calculates points based on the use information and presents information (for example, points, a service restored to the user according to the points, the amount of dividend, or the like) relating to the calculated points to the user.

For example, the incentive processing unit 122a presents public advertisement information to a portal site 14h of user A. In this public advertisement information, for example, an overview of the purpose of the secondary use "Please help for the development of a drug", the value of the point", the data amount (for example, the transmission frequency, the transmission period, and the like) and the kind (for example, specific items of biological information or behavior information) of PHR data required for the purpose are described. For example, user A reads this public advertisement information and goes through the procedure of the application (use permission). In addition, user A transmits the PHR data to the PHR processing apparatus 100 in accordance with the rules of the PHR data that are described in the public advertisement information.

The PHR data of user A that is transmitted to the PHR processing apparatus 100 in this way and is accumulated therein is provided for the secondary use having the purpose described above according to the use permission. Then, as described above, the incentive processing unit 122a receives the use information from the secondary use service providing unit 123, calculates points based on the use information, and presents information relating to the calculated points to the user. For example, the incentive processing unit 122a, as illustrated in FIG. 19, displays "Acquired points of user A are OOO pts" at a portal site 14i. Here, the calculation of points does not necessarily need to be performed after the secondary use but may be performed before the provision of the PHR data for the secondary use.

This point system is operated and managed by the data trust company 11, and the data trust company 11 performs specific restoration based on the points for user A. For example, in a case where the data trust company 11 acquires earnings in relation with the secondary use service, the data trust company 11 affiliates with a company or a store based on the earnings and allows user A to restore points from a company that is an affiliation or a product. The restoration of points, for example, may be in any form such as a service or a free gift. Alternatively, the data trust company 11 may transfer a part of the earnings to user A in the form of dividend.

The points are described to be calculated based on the usefulness of the secondary use service. This point will be additionally described. As the purpose of the secondary use service, there are a purpose that has high social significance as in the case of a clinical test (clinical trial) performed for the development of a drug or the acquisition of an approval defined in the Pharmaceutical Affairs Law and a purpose for a part of simple marketing as in the case of collecting biological information represented in the body from a viewer of a movie or a program. While a purpose to be determined as a purpose having a high degree of usefulness and a purpose to be determined as a purpose having a low degree of usefulness may be arbitrary set by the data trust company 11 side, for example, the data trust company 11 may set the usefulness such that a medical purpose has a high degree of usefulness, and a marketing purpose has a low degree of usefulness. Alternatively, the data trust company 11 may set the usefulness based on a ratio of earnings (or prospected earnings to be acquired) that are actually acquired to total earnings.

In addition, in the example described above, on the premise that the PHR data transmitted from the user is provided also for the secondary use service, while the feedback mechanism for the user such as the point system in any form in relation with the secondary use service has been described, the embodiment is not limited thereto. The point system operated by the incentive processing unit 122a, separately from the secondary use service, may be operated based on only the data amount, the kinds, and the number of kinds of the PHR data transmitted from the user. In such a case, for example, the incentive processing unit 122a calculates points for the PHR data of each user based on at least one of the data amount, the kinds, and the number of the kinds and presents information relating to the calculated points to the user.

Furthermore, in the example described above, while an example has been described in which the points are calculated based on the data amount, the kinds, and the number of the kinds of the PHR data, the usefulness of the secondary use service for which the PHR data is utilized, and the like, the embodiment is not limited thereto. The incentive processing unit 122a may calculate the points based on a criterion used for evaluating the transmission status of the PHR data.

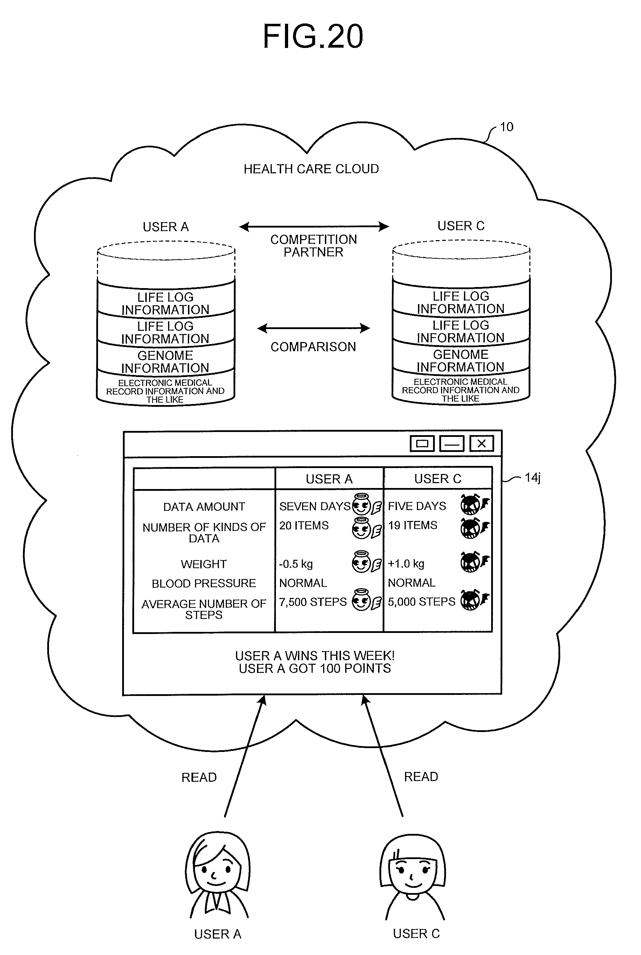

Next, FIG. 20 is a diagram that illustrates a second one of the incentive mechanisms according to this embodiment. Also in the second one, the incentive processing unit 122a evaluates the PHR data of a specific user and presents a result of the evaluation to the specific user. For example, as illustrated in FIG. 20, the incentive processing unit 122a sets a competition relation among a plurality of users transmitting the PHR data and compares the life log information among the users. Then, the incentive processing unit 122a presents a result thereof to the users having the competition relation.

For example, the incentive processing unit 122a accepts an application for setting user C as a competition partner from user A. Then, the incentive processing unit 122a sets a portal site 14j used for a competition between user A and user C on the health care cloud 10 and attaches a link toward this portal site 14j used for the competition to the portal sites of user A and user C. In this way, each of user A and user C can read the portal site 14j used for the competition between two users through the portal site thereof.

In addition, for example, the incentive processing unit 122a updates the portal site 14j used for the competition between two users at a frequency (for example, once a day, once a week, once a month, once a year, or the like) according to the desire of user A and user C. For example, it is assumed that the desire of user A and user C is the frequency of once a week. Then, the incentive processing unit 122a, once a week, extracts information of a competition item designated as a competition target in advance from the PHR data of user A corresponding to the past one week and similarly, extracts information of a competition item designated as a competition target in advance from the PHR data of user C. Then, the incentive processing unit 122a compares the information of the competition item extracted from each PHR data and determines a winner.

In the case of the example illustrated in FIG. 20, for example, the incentive processing unit 122a, for the PHR data of each user, specifies the data amount and the number of the kinds and acquires weight information, blood information, an average number of steps per day, and the like from the life log information. In the example illustrated in FIG. 20, during one week, user A transmits the PHR data seven days and transmits 20 items. User A succeeds in decreasing the weight of 0.5 kg, has normal blood pressure, and has an average number of steps of 7,500 per day, which is a relatively large. On the other hand, user C, during one week, transmits the PHR data five days and transmits 19 items. In addition, user C gains a weight of 1.0 kg, has normal blood pressure, and has an average number of steps of 5,000 per day, which is relatively small.

The incentive processing unit 122a evaluates such information based on a criterion determined in advance and determines a winner. Then, the incentive processing unit 122a, for example, as illustrated in FIG. 20, displays a result of the determination using a mark that visually represents a winner, character information, or the like. In the example illustrated in FIG. 20, the incentive processing unit 122a gives points also to the result of such a competition. The competition items, the criterion of the competition, the GUI of the portal site 14j used for a competition, a method of feeding back the result of the competition, and the liked described above may be arbitrarily changed. For example, the incentive processing unit 122a may be configured to transmit the result of the competition to the mail address of each user.

### (Analysis of Biological Information)

Until now, the example in which the user's health risk is estimated by using the genome information and the life log information collected on a daily basis and the like have been described. However, the use of the genome information and the life log information is not limited to the example described above and may be used for various analyses. For example, there may be a case where the analysis is performed focusing on the biological information associated with the behavior information in the life log information. As described above, as the biological information that is collected from each user day by day, there are blood pressure, a heart rate, a pulse count, a body temperature, a body component, ions, pH concentration, the amounts of components such as sugar and salt, the concentration of gastric acid, presence/no-presence of agrochemical, an environmental material, and a food additive, alcohol, nicotine, a medicament component, and the like. In addition, as the biological information, there is also a bio marker such as protein, peptide, lipid, a sugar chain, and the like.

Here, the bio marker described above, for example, is protein, peptide, lipid, a sugar chain, or the like contained in a body fluid such as a serum, urine, tear, saliva, a cerebrospinal fluid, pancreatic juice, a synovial fluid, mammary gland aspirate, bile, or the like and reflects the physiological condition of the inside of the body. Accordingly, for example, by using a bio marker relating to a disease, the disease can be detected at an early stage, and early treatment can be performed. Information such a bio marker, for example, may be collected by a biological sensor or through a periodic inspection.

As examples of the bio marker, for example, there are "total protein", "GOT (glutamic oxaloacetic transaminase)", "GPT (glutamate pyruvate transaminase)", "ALP (alkaline phosphatase)", "γ-GTP (γ-glutamate pyruvate transaminase)", "LDH (lactic acid dehydrogenase)", "total bilirubin", "amylase", "total cholesterol", "neutral fat", "HDL cholesterol", "hemoglobin", and the like. The bio markers described here are merely examples, and the bio marker as the biological information is not limited thereto. At present, by using various body fluids, searches for bio markers relating to diseases have been actively performed, and various bio markers have been identified. In addition, it is assumed that a plurality of bio markers will be identified from now on. As bio markers according to this embodiment, all the bio markers described above may be used.

Such various kinds of biological information is collected day by day as a user spends everyday life and is accumulated as the PHR big data. The PHR processing apparatus 100 performs various analyses of each biological information and provides results thereof for the user and the like. For example, the PHR processing apparatus 100, for each kind of biological information, compares collected data and derives a result of the comparison. For example, the PHR processing apparatus 100 compares data (for example, blood pressure or the like) of the biological information collected on a daily basis with data of the other persons for an analysis or compares data of individuals for an analysis. Hereinafter, such comparison/analysis will be described.

First, a case will be described in which data is compared between different persons for an analysis. In a case where data is compared between different persons for an analysis, the PHR processing apparatus 100, for example, compares the biological information of a predetermined user with the biological information of the other users and analyzes a result of the comparison. In addition, the PHR processing apparatus 100 divides a plurality of users into groups and compares the biological information of each group with the biological information of the other groups and analyzes a result of the comparison. Here, the PHR processing apparatus 100 divides a plurality of users into groups based on the genome information and attribute information and performs a comparison therebetween.

For example, in a case where the biological information of a predetermined user is compared with the biological information of the other users for an analysis, the PHR processing apparatus 100 divides a plurality of users into groups based on the genome information and the attribute information and compares the biological information among the users inside the group for an analysis. On the other hand, for example, in a case where the biological information of each group is compared with the biological information of the other groups for an analysis, the PHR processing apparatus 100 divides a plurality of users into groups based on the genome information and the attribute information, and the biological information is compared among the groups for an analysis. In the example described below, while a case will be described as an example in which the PHR big data analyzing unit 121 includes a function of a comparison unit that compares the biological information and a function of an analysis unit that analyses a position based on a result of the comparison, the embodiment is not limited thereto. Thus, the PHR processing apparatus 100 may be configured to include a comparison unit and an analysis unit.

More specifically, in a case where the biological information of a predetermined user is compared with the biological information of the other users for an analysis, the PHR big data analyzing unit 121, based on the information accumulated in the PHR accumulation unit 110, compares predetermined biological information with the biological information of each user, which is continuously collected, inside the group formed by combining the genome information and the attribute information of each user. Then, the PHR big data analyzing unit 121, based on a result of the comparisons, represents a position of the value of the biological information of each user inside the group. On the other hand, in a case where the biological information of each group is compared with the biological information of the other groups for an analysis, the PHR big data analyzing unit 121, based on the information accumulated in the PHR accumulation unit 110, for each group acquired by combining the genome information and the attribute information of each user, integrates the biological information of each user belonging to the group, which is continuously collected, and compares the biological information among the groups. Then, based on a result of the comparisons, the PHR big data analyzing unit 121 represents a position of the value of the biological information of each group among the groups.

Here, the PHR big data analyzing unit 121 represents a position in a distribution of values of the biological information as the position. In other words, the PHR big data analyzing unit 121 derives a position of the value of each user in the distribution of values of the predetermined biological information of users belonging to the group as the positions of the users and derives a position of an average value of the groups in the distribution of average values of the values of the predetermined biological information of each group as the position of each group. Here, in the attribute information described above, information of the residential area, the age, the sex, and the race of the user, and the health insurance association to which the user belongs is included, and the PHR big data analyzing unit 121 forms a group acquired by combining the genome information of each user and the above-described attribute information as the group. In addition, in the position described above, a ranking, a deviation value, and the like are included, and the PHR big data analyzing unit 121 derives the ranking or the deviation value of a user inside the group or the ranking, the deviation value, or the like of each group as the position.

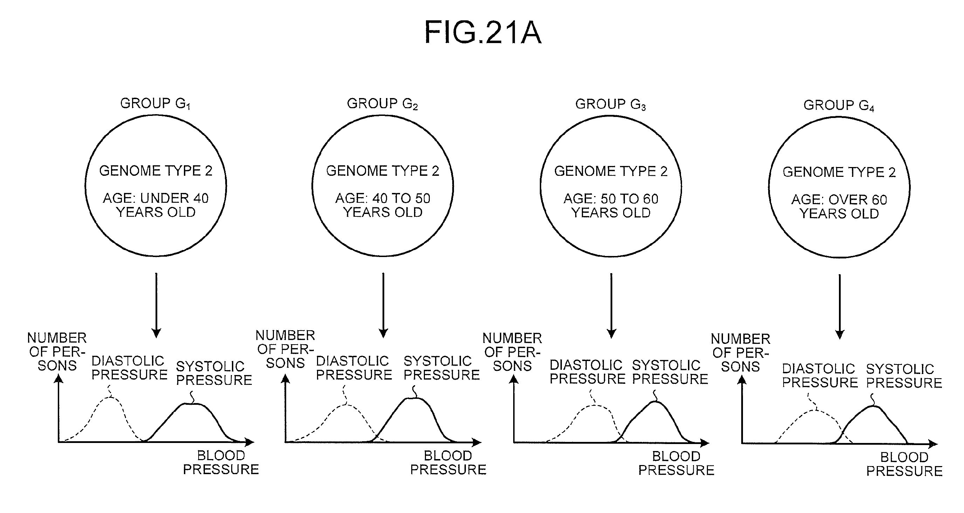

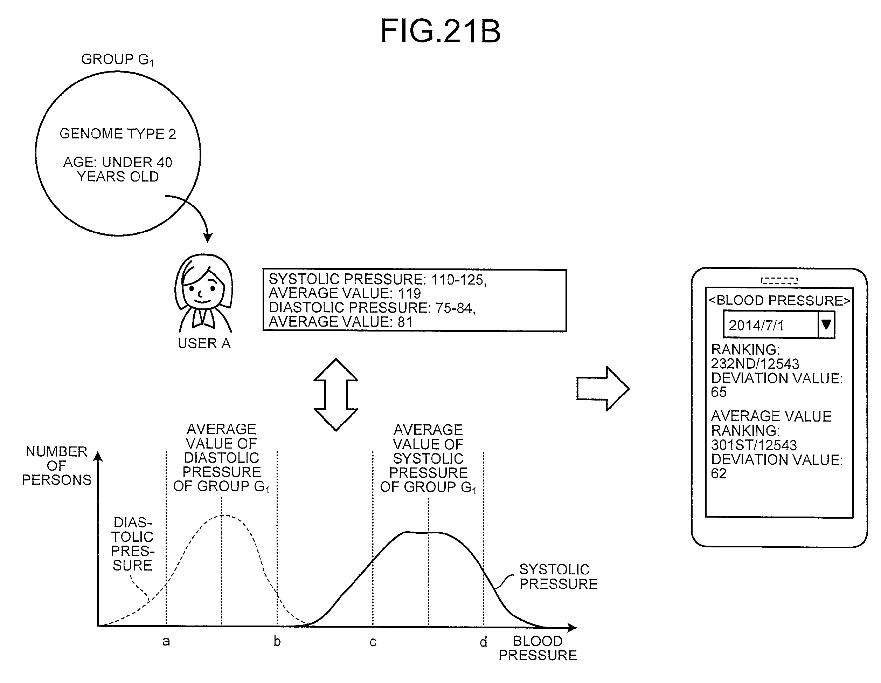

FIGS. 21A and 21B are diagrams that illustrate a comparative analysis between users according to this embodiment. For example, the PHR big data analyzing unit 121, as illustrated in FIG. 21A, classifies a plurality of users into groups G₁ to G₄ according to "genome information: genome type 2" and "attribute information: age". Then, the PHR big data analyzing unit 121, for each classified group, for example, compares blood pressure and represents a position of the value of the blood pressure of each user within the group. For example, the PHR big data analyzing unit 121 integrates information of blood pressure (diastolic pressure and systolic pressure) of all the users belonging to the group G₁ of "genome type 2 and age: up to 40 ages" and, as illustrated in FIG. 21A, generates a histogram for each of the diastolic pressure and the systolic pressure, and derives positions of the blood pressure of users belonging to the group G₁.

Similarly, the PHR big data analyzing unit 121, as illustrated in FIG. 21A, for the group G₂ of "genome type 2 and age: 40 to 50 years old", the group G₃ of "genome type 2 and age: 50 to 60 years old", and the group G₄ of "genome type 2 and age: 60 years and above", generates a histogram of each blood pressure and derives a position of the blood pressure of each user for each group. Here, in FIG. 21A, while the group formed according to "genome information: genome type 2" and "attribute information: age" is illustrated, this is merely an example. Thus, also for the other genome types, similarly, the PHR big data analyzing unit 121 divides a plurality of users into groups according to the type of genome and the age, compares the biological information for each group, and represents a position of the biological information of each user within the group. In addition, as the attribute information, not only the age but also the sex, the residential area, or the like is used. For example, the PHR big data analyzing unit 121 forms a group of "genome information: genome type 2 and attribute information: male", a group of "genome information: genome type 2 and attribute information: female", and the like.

In addition, the attribute information used for the group division is not limited to one, and two or more attribute information may be used. For example, the PHR processing apparatus 100 generates a group of "genome type 2" in a "predetermined residential area" for each "age". As above, the PHR big data analyzing unit 121 divides users into groups by using the "genome information" and the "attribute information" and performs a comparative analysis among users for each group. In this way, the user can check his position in comparison with the other users having the same genome type and attribute information as the user. Hereinafter, an example of the comparative analysis will be described with reference to FIG. 21B. In FIG. 21B, a comparative analysis of the group G₁ illustrated in FIG. 21A is illustrated as an example.

For example, the PHR big data analyzing unit 121, as illustrated in FIG. 21B, derives a position of the measured value of the blood pressure of user A belonging to the group G₁ in the histogram of the group G₁. Here, the histogram of the group G₁ may be acquired by integrating the blood pressure values of users at one time point or may be acquired by integrating average values for a predetermined period. For example, the histogram may be acquired by integrating blood pressure values of all the users belonging to the group G₁ at the rising time of a certain day, or the histogram may be acquired by averaging blood pressure values of all the users belonging to the group G₁ at the rising time corresponding to one week and integrating the average value of each user. Here, it is determined whether or not the user has risen based on the heart rate or the pulse rate measured by a biological sensor worn by the user, and the blood pressure at the rising time is blood pressure measured at a time point at which the user is determined to have risen.

For example, the PHR big data analyzing unit 121, as illustrated in FIG. 21B, derives the position of the blood pressure value "systolic pressure: 110 to 125, average value 119" and "diastolic pressure: 75 to 84, average value 81" of user A corresponding to one week in the histogram acquired by integrating the average value of each user belonging to the group G₁. In other words, the PHR big data analyzing unit 121 derives the position of the average value of the systolic pressure of user A in the histogram of the systolic pressure and derives the position of the average value of the diastolic pressure of user A in the histogram of the diastolic pressure. Here, the PHR big data analyzing unit 121 not only derives the position of user A in the histogram but also compares a reference value arranged for each group and the measured value of each user belonging to the group with each other.

The histogram of each group is acquired by integrating the biological information of users having the same type of genome and the same attribute information. Accordingly, on the distribution state of the histogram, the type of each genome and the attribute information are considered to be reflected. Thus, an average value of the histogram of each group is set as an ideal value (reference value) of the group, and, based on the degree of deviation from the reference value, the users are ranked, and deviation values are given. For example, the PHR big data analyzing unit 121 calculates a difference between the "average value: 119" of the systolic pressure of user A and an average value (reference value) of the systolic pressure of the group G₁. Similarly, the PHR big data analyzing unit 121 calculates a difference between the "average value: 81" of the diastolic pressure of user A and an average value (reference value) of the diastolic pressure of the group G₁. Then, the PHR big data analyzing unit 121 derives the degree of deviation based on the differences calculated for the systolic pressure and the diastolic pressure. For example, the PHR big data analyzing unit 121 derives a sum of the absolute values of the two differences as the degree of deviation.

Similarly, the PHR big data analyzing unit 121 derives the degree of deviation for all the users belonging to the group G₁ and performing ranking user A, assignment of a deviation value, and the like in the group G₁ based on the derived degree of deviation. For example, the PHR big data analyzing unit 121 performs the ranking and the assignment of the deviation value such that a user having a lower degree of deviation from the reference value (the average value of the histogram) is ranked to be higher. In this way, when the PHR big data analyzing unit 121 performs the ranking and the assignment of the deviation value, the primary use service providing unit 122 provides results thereof to each user.

For example, the primary use service providing unit 122, as illustrated in FIG. 21B, provides information of the ranking of the blood pressure and the deviation value for a mobile terminal that is the PHR display apparatus 200 of user A. Here, when the PHR big data analyzing unit 121 derives the ranking and the deviation value of the blood pressure at one time point (for example, at the rising time) and the ranking and the deviation value of an average value of the blood pressure for one week, the primary use service providing unit 122, as illustrated in FIG. 21B, provides a result "ranking: 232/12543 and deviation value: 65" at the one time point and a result "ranking: 301/12543 and deviation value: 62" of the average value for the mobile terminal of user A. The display control unit 210 of the mobile terminal, as illustrated in FIG. 21B, displays the information provided for the display unit 220. In addition, user A can arbitrarily select a result of a desired date.

In addition, the PHR big data analyzing unit 121 not only can perform the ranking and the assignment of the deviation value based on the reference value as described above but also can determine whether or not the measured value of the user is within the range of allowed values. For example, the PHR big data analyzing unit 121 sets an allowed range "a to b" of the diastolic pressure and an allowed range "c to d" of the systolic pressure illustrated in FIG. 21B and determines whether or not the measured values of user A are respectively within the allowed ranges. The primary use service providing unit 122 provides a result of the determination for the mobile terminal of user A. The display control unit 210 of the mobile terminal displays the result of the determination on the display unit 220.

Here, as the allowed ranges set by the PHR big data analyzing unit 121, for example, specified values arranged by various academic societies, groups, organizations, or the like may be used. In addition, the allowed ranges set by the PHR big data analyzing unit 121 may be set based on the histograms. For example, the PHR big data analyzing unit 121 sets an arbitrary range based on a minimal value and a maximal value of the histogram. As an example, the PHR big data analyzing unit 121, among all the numbers of users, counts from the minimal value in the ascending order and sets a value arriving at 30% of the number of the users as a lower limit. In addition, the PHR big data analyzing unit 121 counts from the maximal value in the descending order and sets a value arriving at 30% as the upper limit.

As described above, the PHR big data analyzing unit 121 forms a group acquired by combining the genome information and the attribute information, compares the biological information within the group, and analyzes a position of the value of the biological information of each user within the group. In addition, it is apparent that the example of the blood pressure described above is merely an example, and the comparative analysis is similarly performed for the other biological information (for example, a heart rate, a pulse count, a body temperature, a body component, ions, pH concentration, the amounts of components such as sugar, salt, and the like, the concentration of gastric acid, presence/no-presence of agrochemical, an environmental material, and a food additive, alcohol, nicotine, and a medicament component, a bio marker, and the like).

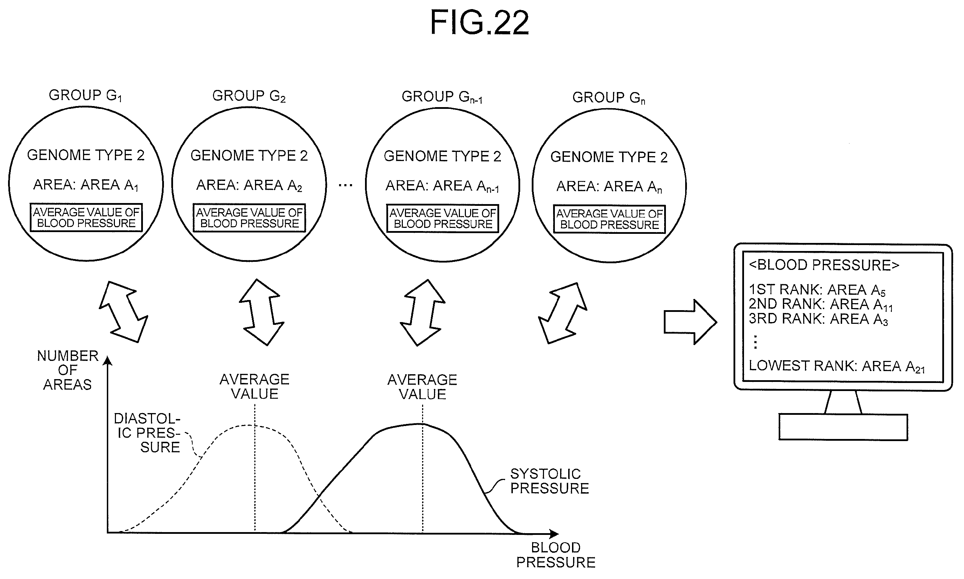

In the example described above, a comparison between users in the comparison between data of different persons has been described. Next, a comparison between groups in the comparison between data of different persons will be described. As described above, the PHR big data analyzing unit 121 divides a plurality of users into groups acquired by combining the genome information and the attribute information. In this embodiment, the PHR processing apparatus 100 performs a comparison between such groups for predetermined biological information. FIG. 22 is a diagram that illustrates a comparative analysis between groups according to this embodiment. Here, in FIG. 22, a case is illustrated in which a plurality of users are divided into groups G₁ to Gₙ based on a combination of "attribute information: genome type 2" and "attribute information: residential area", and the blood pressure is compared.

For example, the PHR big data analyzing unit 121, as illustrated in FIG. 22, calculates an average value of the blood pressure (the diastolic pressure and the systolic pressure) for the groups G₁ to Gₙ. Then, the PHR big data analyzing unit 121 generates histograms by using the average value of the diastolic pressure and the average value of the systolic pressure calculated for each group and derives a position of the average value of each group in the histogram. For example, the PHR big data analyzing unit 121 sets the average value of the histogram as a reference value and performs ranking of each group and assignment of the deviation value based on the degree of deviation from the reference value. For example, the PHR big data analyzing unit 121 calculates a difference between an average value of the systolic pressure of the group G₁ and an average value of the systolic pressure of all the groups. Similarly, the PHR big data analyzing unit 121 calculates a difference between an average value of the diastolic pressure of the group G₁ and an average value of the diastolic pressure of all the groups. Then, the PHR big data analyzing unit 121 derives the degree of deviation based on the differences calculated for the systolic pressure and the diastolic pressure. As an example, the PHR big data analyzing unit 121 derives a sum of the absolute values of the two differences as the degree of deviation.

Similarly, the PHR big data analyzing unit 121 derives the degree of deviation for each of all the groups G₂ to Gₙ and performs ranking of the groups, the assignment of deviation values, and the like based on the derived degrees of deviation. For example, the PHR big data analyzing unit 121 performs the ranking and the assignment of deviation values such that a group having a lower degree of deviation from the reference value (the average value of the histogram) is ranked to be higher. In this way, when the PHR big data analyzing unit 121 performs the ranking and the assignment of deviation values, the primary use service providing unit 122 or the secondary use service providing unit 123 provides the results for the user, a company, an organization, and the like.

For example, the secondary use service providing unit 123, as illustrated in FIG. 22, provides the information of the blood pressure ranking for terminals of a company, an organization, and the like desiring the results. A user referring to the terminal with which the information is provided can check that "area A₅" is ranked first and "area A₂₁" is ranked last for the blood pressure. Accordingly, for example, for each region, a best measure can be taken. In addition, as is apparent, the example illustrated in FIG. 22 is merely an example, each group is formed by arbitrarily combining the genome information and the attribute information, arbitrary biological information is compared, and the position of each group among the plurality of groups is represented. In addition, the average value calculated for each group may be an average value of values at one time point, or it may be configured such that an average value of a predetermined period is calculated for each user, and an average value of the calculated average values is set as the average value calculated for each group.

In the example described above, a case has been described in which data between different persons is compared for an analysis. Next, a case will be described in which data between persons is compared for an analysis. In a case where data between persons is compared for an analysis, the PHR processing apparatus 100 analyzes a trend of the measured value of the biological information of a predetermined user. For example, the PHR processing apparatus 100 analyzes the biological information at a specific time point and analyzes a trend of the change in the biological information in the width of the predetermined period. Here, the PHR processing apparatus 100 analyzes the measured value with respect to the reference value derived based on the genome information.

More specifically, the PHR big data analyzing unit 121, for predetermined biological information, derives a result of a comparison between the reference value derived based on the genome information and the measured value collected from the user corresponding to the genome information. Furthermore specifically, the PHR big data analyzing unit 121 derives at least one of a result of a comparison between the reference value and the measured value at a predetermined time point and a result of a comparison between a tendency of a change in the measured value collected from the user for the width of a predetermined period and a tendency of a change in the reference value corresponding to the width of the predetermined period.

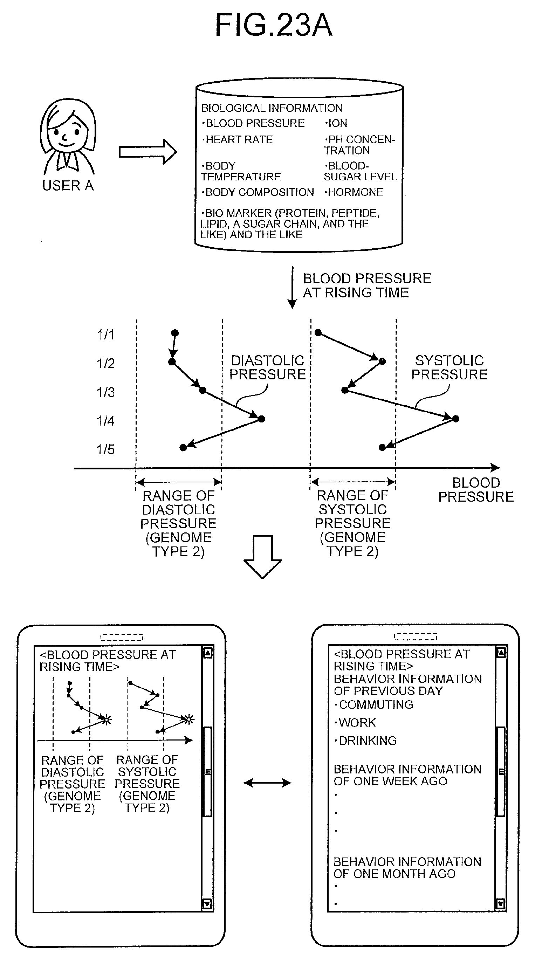

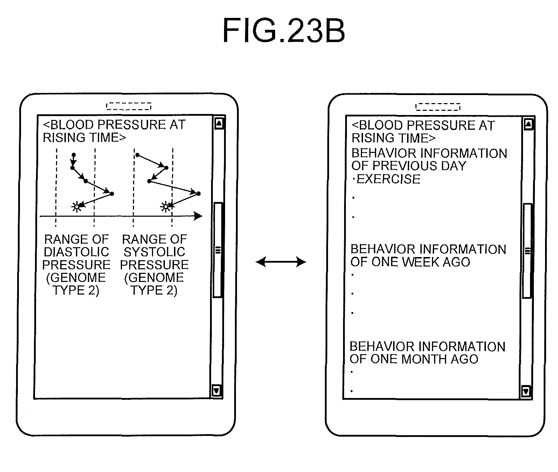

Hereinafter, the comparative analysis between a reference value and a measured value at a predetermined time point will be described. In such a case, the PHR big data analyzing unit 121, first, sets a reference value based on the genome information of the user that is a target for the analysis. Then, the PHR big data analyzing unit 121, for the set reference value, derives a measured value of the user. FIGS. 23A and 23B are diagrams that illustrate a comparative analysis of a predetermined time point of an individual according to this embodiment. For example, as illustrated in FIG. 23A, in a case where a comparative analysis is performed for the blood pressure at the rising time of user A of "genome type 2", the PHR big data analyzing unit 121, first, sets a reference value based on genome type 2.

As described above, a value acquired by integrating measured values of users of the same genome type is a value on which the type of the genome is reflected and may be considered as a reference value. Thus, for example, the PHR big data analyzing unit 121 acquires data of the blood pressure (the diastolic pressure and the systolic pressure) at the rising time of the other users of genome type 2 from the PHR accumulation unit 110, generates a histogram, and sets an arbitrary range based on a minimal value and a maximal value of the histogram. For example, the PHR big data analyzing unit 121, among the number of the other users of genome type 2, counts from the minimal value in the ascending order and sets a value arriving at the number of users of 30% as a lower limit. In addition, the PHR big data analyzing unit 121 counts from the maximal value in the descending order and sets a set range having a value arriving at 30% as an upper limit as a reference value. The PHR big data analyzing unit 121 sets a "diastolic pressure range (genome type 2)" and a "systolic pressure range (genome type 2)" illustrated in FIG. 23A by using the technique described above.

Then, the PHR big data analyzing unit 121, as illustrated in FIG. 23A, compares the value of the blood pressure of user A measured at the rising time with the set range. In other words, the PHR big data analyzing unit 121 analyzes whether or not the diastolic pressure at the rising time of user A fits into the diastolic pressure range (genome type 2). Similarly, the PHR big data analyzing unit 121 analyzes whether or not the systolic pressure at the rising time of user A fits into the systolic pressure range (genome type 2). The PHR big data analyzing unit 121 performs the above-described analysis for the blood pressure at the rising time that is measured on a daily basis. Here, the setting of a reference value may be performed for each analysis or may be performed every time when a constant period elapses.

In this way, when the comparative analysis is performed by the PHR big data analyzing unit 121, the primary use service providing unit 122 provides the result for each user. For example, the primary use service providing unit 122, as illustrated in FIG. 23A, provides a result of a comparison between a trend of daily blood pressure and the reference value (range) for a mobile terminal of user A. The display control unit 210 of the mobile terminal displays the provided result of the comparison on the display unit 220. Here, in a case where the measured value deviates from the range, the display control unit 210, as illustrated in FIG. 23A, emphasizes a plot representing the deviating measured value by coloring the plot or blinking the plot. Accordingly, user A can check the measured value deviating from the reference value at first sight.

In addition, when user A performs a touch operation or the like for the plot representing the measured value deviating from the reference value, the mobile terminal communicates with the PHR processing apparatus 100, and the primary use service providing unit 122 provides behavior information "(commuting, work, and drinking)" of the previous day, behavior information of one week ago, behavior information of one month ago, and the like of user A. In other words, the display control unit 210, as illustrated in FIG. 23A, displays the information on the display unit 220. Accordingly, user A can estimate the reason for the deviation of the measured value from the reference value and thereafter can live while paying attention to the estimated reason.

Here, the measured values provided for the user in an emphasized manner may be not only a value deviating from the reference value (range) but also a value fitting into the reference value. For example, the display control unit 210, as illustrated in FIG. 23B, emphasizes on a plot representing a measured value of a day fitting into the reference value and a measured value of the previous day deviating from the reference value by coloring the plot or blinking the plot. Accordingly, user A can check the measured value fitting into the reference value at first sight. In addition, when user A performs a touching operation or the like, the mobile terminal communicates with the PHR processing apparatus 100. The primary use service providing unit 122 provides behavior information "exercise" of the previous day, behavior information of one week ago, behavior information of one month ago, or the like of user A. In other words, the display control unit 210, as illustrated in FIG. 23B, displays the information on the display unit 220. Accordingly, user A can estimate the reason why the measured value converges into the reference value.

In the above-described example, while an example has been described in which the range is used as the reference value, one value may be used as the reference value. For example, there may be a case where the PHR big data analyzing unit 121 uses the average value of the blood pressure (the diastolic pressure and the systolic pressure) at the rising time of the other users of genome type 2 as the reference value. In such a case, for example, in a case the measured value is above the reference value (or below the reference value), the display control unit 210 displays a plot representing the measured value in an emphasized manner.

In the example described above, while a case has been described in which a measured value of one time point of one day is analyzed, it may be configured such that measurement is performed a plurality of times on one day, and the analysis is performed for each time point. For example, in a case where the body temperature is measured a plurality of times on one day, the analysis may be performed for each measured value. In addition, timing at which the result is provided for the user may be arbitrarily set. For example, the primary use service providing unit 122 may provide the result simultaneously when the "future health risk notification" described above is provided, or the result may be provided for the user every time when the analysis is performed. In addition, the result may be provided for the user in a case where the measured value deviates from the reference value (range), a case where the measured value is above the reference value, or a case where the measured value is below the reference value. Accordingly, for example, when the body temperature changes markedly, the change in the body temperature can be instantly notified to the user.

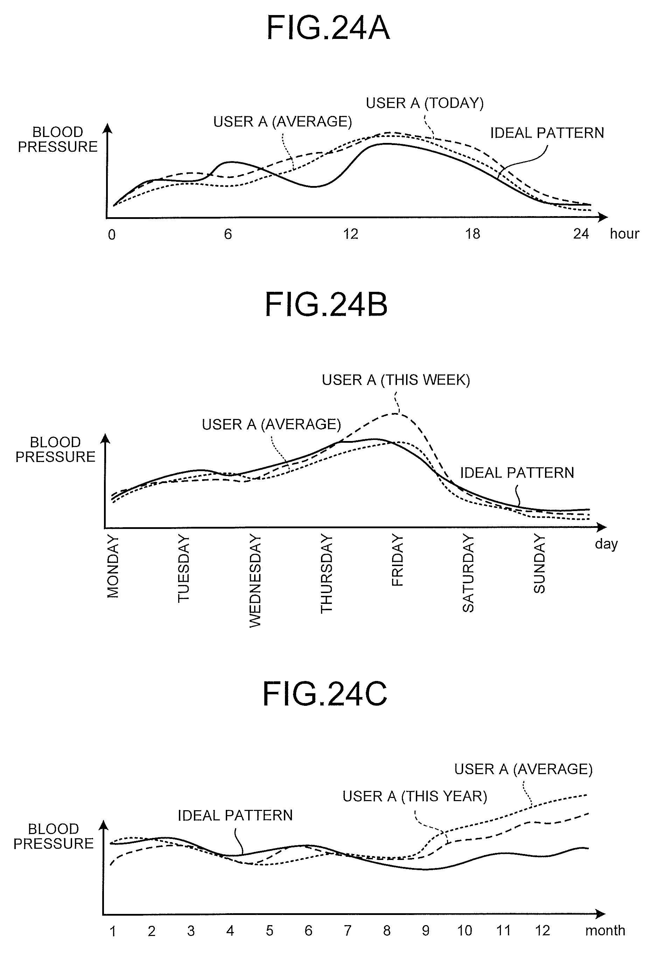

Next, the comparison between the tendency of a change in the measured value collected from the user for a width of a predetermined period and the tendency of a change in the reference value corresponding to the width of the predetermined period will be described. Also in such a case, the PHR big data analyzing unit 121, first, sets the tendency of a change in the reference value based on the genome information of the user that is an analysis target. Then, the PHR big data analyzing unit 121 derives the tendency of a change in the measured value of the user with respect to the tendency of a change in the set reference value. FIGS. 24A to 24C are diagrams that illustrate a comparative analysis of an individual for a predetermined period according to this embodiment. Here, in FIGS. 24A to 24C, a case is illustrated in which the tendency of a change in the systolic pressure of user A of "genome type 2" is analyzed. In addition, FIG. 24A illustrates a case where the predetermined period is one day, FIG. 24B illustrates a case where the predetermined period is one week, and FIG. 24C illustrates a case where the predetermined period is one year.

For example, the PHR big data analyzing unit 121 acquires measured values of the systolic pressure that are collected over time from a plurality of users of genome type 2 from the PHR accumulation unit 110 and calculates an average value for each hour. Then, the PHR processing apparatus 100 sets the tendency of a change in the average value of each hour as the tendency of the change in the reference value. For example, the PHR big data analyzing unit 121, as illustrated in FIG. 24A, for a plurality users of genome type 2, sets the tendency of a change in the average value of the systolic pressure collected over time from 0 o'clock to 24 o'clock as an ideal pattern. Then, the PHR big data analyzing unit 121 derives a result of a comparison between the tendency of the change in the measured value of user A from 0 o'clock to 24 o'clock and the ideal pattern.

For example, the primary use service providing unit 122 provides the result of the comparison that is derived by the PHR big data analyzing unit 121 for the mobile terminal of user A as a graph as illustrated in FIG. 24A. The display control unit 210 of the mobile terminal displays the provided result of the comparison on the display unit 220. Accordingly, user A can check that today's systolic pressure of the user has a trend of being slightly higher than the ideal pattern by referring to today's measured value "user A (today)" and the "ideal pattern" illustrated in FIG. 24A and recognizes that it is necessary to pay attention to the blood pressure.

In addition, the PHR processing apparatus 100 can derive not only the tendency of today's measured value but also the tendency of an average value acquired by averaging measured values of one day measured by user A in the past for each hour. In such a case, the primary use service providing unit 122, as illustrated in FIG. 24A, provides a graph of "user A (average)" for user A in addition to the today's measured value "user A (today)" and the "ideal pattern". Accordingly, user A can check the status of the value of today's systolic pressure in comparison with a normal value at first sight. In addition, user A can check the state of the tendency of the systolic pressure of one day of the user in comparison with the ideal pattern at first sight. For example, user A views the graph illustrated in FIG. 24A and can check not only today's systolic pressure but also the trend of the blood pressure being usually slightly high, and accordingly, the user can take a countermeasure in a speedy manner.

Similarly, for example, as illustrated in FIGS. 24B and 24C, the PHR big data analyzing unit 121 derives the trend of the systolic pressure of one week and the tendency of the systolic pressure of one year of user A, and the primary use service providing unit 122 provides graphs thereof for user A. Thus, user A can check the transition of his systolic pressure user for each period at first sight by referring to the graphs illustrated in FIGS. 24B and 24C. For example, user A refers to the graph illustrated in FIG. 24B and can consider that, while Friday's systolic pressure tends to be high during a week, the systolic pressure becomes high also in the ideal pattern, and thus he does not need to worry about it. In addition, user A refers to the graph illustrated in FIG. 24C, and the systolic pressure tends to be high in the winter during a year and is different from that of the ideal pattern, and thus, the user recognizes that it is necessary to pay attention to the blood pressure of the winter season. In addition, it is apparent that the examples illustrated in FIGS. 23A, 23B, 24A, and 24B are merely examples, and the comparative analysis is performed for arbitrary biological information.

As above, the PHR big data analyzing unit 121 derives the comparison result acquired by comparing the tendency of a change in the biological information for periods of various widths with the ideal pattern or the tendency of a change in the measured value in the past. The user refers to the comparison result and lives such that the tendency of the change for each period approaches the ideal pattern for various kinds of biological information, thereby causing the body state to be a more ideal state. In addition, the timing at which the comparison result described above is presented may be arbitrarily set. For example, the primary use service providing unit 122 may provide the result simultaneously when the "future health risk notification" described above is provided or may provide the result for the user every time when a predetermined period elapses.

In addition, in the example described above, while a case has been described in which a reference value (a range, an average value, an ideal pattern, or the like) is set by using measured values of a plurality of users having the same type of genome, the reference value may be set by combining the measured values of the user and defined values arranged by various societies, groups, organizations, or the like. Furthermore, the PHR big data analyzing unit 121 may derive a result of a comparison between a value derived based on user's attribute information and a measured value together with the comparison result acquired by comparing the reference value and the measured value with each other. For example, the PHR big data analyzing unit 121, in the comparison of the blood pressure at the rising time illustrated in FIG. 23A, in addition to the range derived from the genome information, may set the range of a defined value arranged by various academic societies, groups, organizations, or the like. In such a case, the primary use service providing unit 122 provides the comparison result in which the range derived from the genome information and the range of the defined value are represented for the user.

Furthermore, in the example described above, while a case has been described in which the primary use service providing unit 122 provides the result of the analysis for the user, the embodiment is not limited thereto, and the result of the analysis may be provided for an attending doctor. In such a case, the attending doctor may refer to the result of the analysis and send a comment to the user.

### (Detailed Configuration of PHR Processing Apparatus 100)

Until now, the "daily medical checkup" service and the "secondary use service" of the PHR data, which are provided in this embodiment, have been described in detail. While the basic configuration of the PHR processing apparatus 100 has been described, hereinafter, the configuration of the PHR processing apparatus 100 will be described in more detail. All the PHR accumulation unit 110, the PHR big data analyzing unit 121, the primary use service providing unit 122, and the secondary use service providing unit 123 to be described below respectively correspond to the PHR accumulation unit 110, the PHR big data analyzing unit 121, the primary use service providing unit 122, and the secondary use service providing unit 123 described in the above-described embodiment. In addition, the PHR processing apparatus 100 does not necessarily include all the units described below, and some of the units may be omitted as is appropriate. Furthermore, the PHR processing apparatus 100 may further include any other unit.

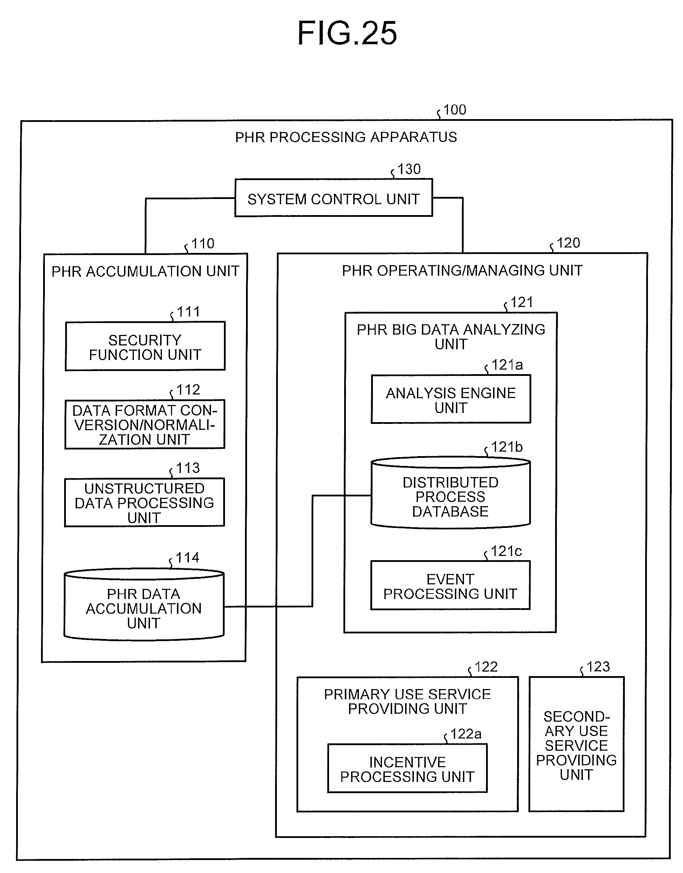

FIG. 25 is a functional block diagram of the PHR processing apparatus 100 according to this embodiment. The PHR processing apparatus 100 can be realized by using one or a plurality of general-purpose computers and includes a processor, a memory, and an input/output interface. Each unit illustrated in FIG. 25 may be appropriately assigned to the processor, the memory, and the input/output interface.

The PHR processing apparatus 100 includes: a PHR accumulation unit 110, a PHR operating/managing unit 120; and a system control unit 130. The system control unit 130 controls the overall operation of the PHR processing apparatus 100. For example, the system control unit 130 receives an operation from an operator of the data trust company 11 and performs account registration of a user who is a management target of the PHR data, family members thereof, and an attending doctor and account registration of medical institutions, various companies, and the like receiving the provision of the secondary use service.

The PHR accumulation unit 110 includes: a security function unit 111; a data format conversion/normalization unit 112; an unstructured data processing unit 113; and a PHR data accumulation unit 114.

The security function unit 111 performs various processes used for acquiring the security of the PHR data. The PHR data is personal information requiring extremely sensitive handling. For this reason, the security function unit 111 performs authentication of a connection destination and approves an access right as the input/output interface (API: Application Programming Interface) of the PHR data. In addition, in order to provide data for utilization and applications after a process for causing each individual not to be specified is performed therefore, the security function unit 111 performs an anonymity process of the PHR data as is necessary. Furthermore, the security function unit 111 encrypts the PHR data for which the anonymity process is not performed using an appropriately managed encryption key. In addition, in a case where the PHR data is to be provided for a destination other than the health care cloud 10, the security function unit 111 performs data transmission having durability against unrightful infringement.

In addition, the security function unit 111 provides a function for performing appropriate personal authentication for all the data access users such as a system supervisor, a researcher analyzing the PHR big data, and an individual user registering PHR data and referring to the registered PHR data. Since sensitive health information of an individual is handled on the health care cloud 10, the security function unit 111 provides a multielement authentication technology for securing security of a level higher than the security level of ID/password authentication. In addition, the security function unit 111 provides a name identification function used for identifying and specifying the owner of data input from various devices and systems.

In order to flexibly respond to the transmission of the PHR data from devices in various forms, the data format conversion/normalization unit 112 provides a data changing/normalizing function and a service bus function for delivering converted normalized data to a predetermined system. In addition, in this embodiment, in order to perform an analysis relating to medical care and health of individuals, the PHR accumulation unit 110 collects complementary information such as information of images and Twitter text of social media or the like and information of audio, images, and texts supplied from a smartphone application. For this reason, the unstructured data processing unit 113 has an interface function and functions called speech recognition, a natural language analysis, image recognition, and data mining used for processing such unstructured data.

The PHR data accumulation unit 114 is the large-scale genome cohort database 114a in which the PHR big data is accumulated.

The PHR operating/managing unit 120 includes: a PHR big data analyzing unit 121; a primary use service providing unit 122; and a secondary use service providing unit 123. In addition, the PHR big data analyzing unit 121 includes: an analysis engine unit 121a; a distributed process database 121b; and an event processing unit 121c. The analysis engine unit 121a performs a cohort analysis and the like of the PHR big data stored in the PHR data accumulation unit 114 as the target. The analysis performed by the analysis engine unit 121a may be performed by using a distributed processing technology. In such a case, the PHR data accumulation unit 114 and the distributed process database 121b cooperate with each other, and the analysis engine unit 121a has the PHR big data stored in the distributed process database 121b as its processing target. In addition, the event processing unit 121c performs an event process according to the distributed process performed by the analysis engine unit 121a.

The primary use service providing unit 122 provides the "daily medical checkup" service as the primary use service. In addition, the primary use service providing unit 122 includes an incentive processing unit 122a. In order for an individual user to wear a sensor and to continue the input of his health information and the supplement information thereof over a long period, an incentive is of significance. The incentive processing unit 122a provides a point system that can be an incentive, various rankings, game elements, and a function of an advertisement model. The secondary use service providing unit 123 provides the secondary use service.

### (Hardware Configuration)

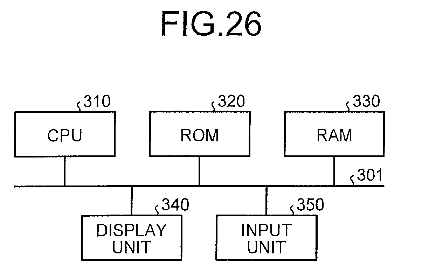

FIG. 26 is a diagram that illustrates the hardware configuration of the PHR processing apparatus 100 (or the PHR display apparatus 200) according to this embodiment. The PHR processing apparatus 100 (or the PHR display apparatus 200) includes: a CPU (Central Processing Unit) 310; ROM (Read Only Memory) 320; RAM (Random Access Memory) 330; a display unit 340; and an input unit 350. In addition, in the PHR processing apparatus 100 (or the PHR display apparatus 200), the CPU 310, the ROM 320, the RAM 330, the display unit 340, and the input unit 350 are interconnected through a bus line 301.

In the embodiment described above, a PHR processing program (or a PHR display program) performing various processes is stored in the ROM 320 and is loaded into the RAM 330 through the bus line 301. The CPU 310 executes the PHR processing program (or the PHR display program) loaded into the RAM 330. For example, in the PHR processing apparatus 100 (or the PHR display apparatus 200), according to the input of an instruction from the input unit 350 that is performed by the operator, the CPU 310 reads the PHR processing program (or the PHR display program) from the inside of the ROM 320, expands the read program into a program storage area arranged inside the RAM 330, and executes various processes. The CPU 310 causes various kinds of data generated at the time of performing these various processes to be temporarily stored in a data storage area formed inside the RAM 330.

The PHR processing program (or the PHR display program) executed by the PHR processing apparatus 100 (or the PHR display apparatus 200) has a module configuration including the PHR big data analyzing unit 121, the primary use service providing unit 122, and the secondary use service providing unit 123 (or the display control unit 210), and such modules are loaded into a main storage device and are generated on the main storage device.

### (Other Embodiments)

The embodiments are not limited to the above-described embodiments.

### (Configuration)

In the above-described embodiment, while a configuration has been described in which the PHR processing apparatus 100 is built on the cloud, the embodiment is not limited thereto. Some or all of the functions of the PHR processing apparatus 100, for example, may be built on a network arranged inside the data trust company 11. In addition, the PHR processing apparatus 100 does not need to be built at one base. The PHR processing apparatus 100 may be realized by cooperating functions that are distributed at a plurality of bases.

In addition, the physical configurations illustrated in the above-described embodiment are merely examples. The units illustrated in the above-described embodiment are appropriately integrated or distributed according to the operating form or the load thereof.

While several embodiments of the present invention have been described, such embodiments are presented as examples but are not for the purposes of limiting the scope of the invention. These embodiments may be performed in various other forms, and various omissions, substitutions, and changes may be made therein without departing from the concept of the invention. When the embodiments and modifications thereof are included in the scope or the concept of the invention, similarly, they belong to the invention defined in claims and the range of equivalents thereof.

## Claims

1. A health information processing apparatus comprising:
an accumulation unit that stores genome information and biological information and behavior information being continuously collected, which are health information of each user, that correspond to a plurality of users;
an analysis unit that analyzes the stored health information corresponding to the plurality of users; and
an estimation unit that estimates a future health risk of a predetermined user by using a result of the analysis and the health information of the predetermined user.

2. The health information processing apparatus according to claim 1, wherein the analysis unit derives relevance between a lifestyle and a health risk derived from the genome information, the biological information, and the behavior information by analyzing the health information corresponding to the plurality of users.

3. The health information processing apparatus according to claim 1 or 2, wherein the analysis unit derives relevance between a combination of a type of genome derived from the genome information and a type of lifestyle derived from the biological information and the behavior information and a diseases having a possibility of being caused in the future by analyzing the health information corresponding to the plurality of users.

4. The health information processing apparatus according to claim 3,
wherein the analysis unit acquires a list of diseases causable in the future as a result of the analysis, the list of the diseases being ordered in accordance with a stronger influence of a genome factor or a lifestyle factor for each combination of a type of genome and a type of lifestyle, and
wherein the estimation unit estimates diseases causable in the future for the predetermined user by referring to the list by using the combination of the type of genome and the type of lifestyle of the predetermined user derived from the health information of the predetermined user.

5. The health information processing apparatus according to claim 1 or 2, wherein, in a case where the future health risk of the predetermined user is estimated, the estimation unit adjusts a result of the estimation in accordance with a health state of the present of the predetermined user, the health state of the present being determined based on the health information of the predetermined user.

6. The health information processing apparatus according to claim 1 or 2,
wherein the accumulation unit increases a scale of the health information corresponding to the plurality of users by newly accumulating the health information,
wherein the analysis unit acquires a result of a new analysis by performing the new analysis in accordance with the increase in the scale of the health information corresponding to the plurality of users, and
wherein the estimation unit estimates the future health risk of the predetermined user by using the result of the analysis that is newly acquired and the health information of the predetermined user.

7. The health information processing apparatus according to claim 1 or 2, wherein the genome information includes at least one of arrangement information of base pairs of about three billion, arrangement information of base pairs of about one million, and a difference from standard genome information.

8. The health information processing apparatus according to claim 1 or 2, wherein the health risk is an outbreak probability of each disease.

9. A health information processing apparatus comprising:
an accumulation unit that stores genome information and biological information and behavior information being continuously collected, which are health information of each user, that correspond to a plurality of users;
an analysis unit that analyzes the stored health information corresponding to the plurality of users; and
a presentation unit that evaluates the health information of a predetermined user that is stored in the accumulation unit and is analyzed by the analysis unit and present a result of the evaluation to the predetermined user.

10. The health information processing apparatus according to claim 9, wherein the presentation unit calculates points based on at least one of a data amount, kinds, and the number of kinds of the health information collected from each user and present information relating to the calculated points to the user.

11. The health information processing apparatus according to claim 10,
wherein the health information is utilized for an estimation of a future health risk and a secondary use having a purpose different from that of the estimation of the future health risk, and
wherein the presentation unit calculates the points also based on usefulness of the secondary use for which the health information is utilized.

12. The health information processing apparatus according to claim 9, wherein the presentation unit sets a competition relation among the plurality of users transmitting the health information, compare the health information among the users to which the competition relation is set, and present a result of the comparison to each user.

13. A health information display apparatus comprising:
a display unit; and
a display control unit that displays a future health risk of a user, which is estimated based on genome information and biological information and behavior information, which are continuously collected, that are health information of the user on the display unit,
the display control unit displays at least one of a target health state of the user and guidance information used for arriving at the target health state together with the future health risk.

14. The health information display apparatus according to claim 13, wherein, when designation of a time point of the estimation is received from an operator, the display control unit displays the future health risk corresponding to the received time point.

15. The health information display apparatus according to claim 13 or 14, wherein, when a width of a period of the health information of the user that is used for the estimation is received from an operator, the display control unit displays the future health risk corresponding to the received width of the period.

16. The health information display apparatus according to claim 13 or 14, wherein, when a change instruction for changing a lifestyle is received from an operator, the display control unit further displays the future health risk simulated in accordance with the received change instruction.

17. The health information display apparatus according to claim 13 or 14, wherein the display control unit displays names of diseases to be caused in the future for the user as the future health risk.

18. The health information display apparatus according to claim 17, wherein, in a case where the names of diseases are displayed for the user or a related person of the user, the display control unit displays the names using common names.

19. The health information display apparatus according to claim 17, wherein, in a case where the names of the diseases are displayed for the user or a related person of the user, the display control unit does not display the names of some of the diseases.

20. A health information display apparatus comprising:
a display unit; and
a display control unit that displays a future health risk of a user, which is estimated based on genome information and biological information and behavior information, which are continuously collected, that are health information of the user on the display unit,
the display control unit that displays the health information of the user together with the future health risk.

21. The health information display apparatus according to claim 20, wherein the display control unit displays a type of genome derived from the genome information and a type of lifestyle derived from the biological information and the behavior information as the health information of the user.

22. The health information display apparatus according to claim 20 or 21, wherein the display control unit displays names of diseases to be caused in the future for the user as the future health risk.

23. The health information display apparatus according to claim 22, wherein, in a case where the names of the diseases are displayed for a medical worker, the display control unit displays each of the names using at least one of a formal name and an ICD (International Classification of Diseases) code.

24. The health information display apparatus according to claim 20 or 21, wherein the display control unit displays a health risk ranking list of a plurality of users based on a comparison with other users other than the user and, in a case where a predetermined user is designated for the health risk ranking list, displays the future health risk and the health information of the designated user.

25. A health information processing apparatus comprising:
an accumulation unit that stores genome information and biological information and behavior information being continuously collected, which are health information of each user, that correspond to a plurality of users;
an analysis unit that derives relevance between a combination of a type of genome derived from genome information and a type of lifestyle derived from biological information and behavior information and a predetermined purpose by receiving an input of the predetermined purpose and analyzing the stored health information corresponding to the plurality of users; and
an output unit that outputs a result of the analysis.

26. The health information processing apparatus according to claim 25,
wherein the analysis unit acquires a classification result acquired by classifying combinations of a type of genome and a type of lifestyle based on the relevance with the predetermined purpose, and
wherein the output unit outputs the classification result as a result of the analysis.

27. The health information processing apparatus according to claim 26, wherein the output unit outputs a real name list of users belonging to a predetermined group acquired as the classification result.

28. The health information processing apparatus according to claim 27, wherein the output unit, first, outputs the real name list of the users belonging to the predetermined group acquired as the classification result and, second, outputs the health information of some of the users belonging to the predetermined group.

29. A health information processing apparatus comprising:
an accumulation unit that stores genome information and biological information and behavior information being continuously collected, which are health information of each user, that correspond to a plurality of users; and
an output unit that receives disclosure items of the health information and use permission of the health information for a disclosure destination from the user and outputs the health information according to a content of the received use permission,
the accumulation unit that stores information of the use permission in accompaniment with the health information.

30. A health information processing apparatus comprising:
an accumulation unit that stores genome information and biological information and behavior information being continuously collected, which are health information of each user, that correspond to a plurality of users;
a comparison unit that compare predetermined biological information with the biological information of each user, which is continuously collected, within a group formed by combining the genome information and attribute information of each user based on the information stored in the accumulation unit; and
an analysis unit that represents a position of a value of the biological information of each user within the group based on a result of the comparison performed by the comparison unit.

31. A health information processing apparatus comprising:
an accumulation unit that stores genome information and biological information and behavior information being continuously collected, which are health information of each user, that correspond to a plurality of users;
a comparison unit that integrates, for each group formed by combining the genome information and attribute information of each user, the biological information, which is continuously collected, of each user belonging to the group based on the information stored in the accumulation unit and compares the biological information between the groups; and
an analysis unit that represents a position of a value of the biological information of each group among the groups based on a result of the comparison performed by the comparison unit.

32. The health information processing apparatus according to claim 30 or 31, wherein the analysis unit represents a position in a distribution of values of the biological information as the position.

33. The health information processing apparatus according to claim 30 or 31, wherein the analysis unit represents a position of the value of the biological information using at least one of a ranking and a deviation value derived using the biological information of each user that is continuously collected.

34. The health information processing apparatus according to claim 30 or 31,
wherein the attribute information includes information of a residential area, age, sex and a race of the user and a health insurance association to which the user belongs, and
wherein the analysis unit forms a group acquired by combining the genome information and the attribute information of each user as the group.

35. A health information processing apparatus comprising:
an accumulation unit that stores genome information and biological information, which is continuously collected, that are health information of each user and correspond to a plurality of users; and
an analysis unit that derives a result of a comparison between a reference value derived based on the genome information and a measured value collected from a user corresponding to the genome information for predetermined biological information.

36. The health information processing apparatus according to claim 35, wherein the analysis unit derives at least one of a result of a comparison between the reference value and the measured value at a predetermined time point and a result of a comparison between a tendency of a change in the measured value collected from the user in a width of a predetermined period and a tendency of a change in the reference value corresponding to the width of the predetermined period.

37. The health information processing apparatus according to claim 35 or 36, wherein the analysis unit derives a result of a comparison between a value derived based on attribute information of the user and the measured value together with the result of the comparison between the reference value and the measured value.

38. A method of processing health information using a health information processing apparatus, the method comprising:
collecting genome information and biological information and behavior information being continuously collected, which are health information of each user, from each user;
storing the health information of each user that corresponds to a plurality of users;
analyzing the stored health information corresponding to the plurality of users;
estimating a future health risk of a predetermined user by using a result of the analysis and the health information of the predetermined user; and
transmitting the future health risk to a health information display apparatus.

39. A method of processing health information using a health information processing apparatus, the method comprising:
collecting genome information and biological information and behavior information being continuously collected, which are health information of each user, from each user;
storing the health information of each user that corresponds to a plurality of users;
analyzing the stored health information corresponding to the plurality of users;
evaluating the analyzed health information of a predetermined user; and
presenting a result of the evaluation to the predetermined user.

40. A method of displaying health information using a health information display apparatus, the method comprising:
displaying a future health risk of a user, which is estimated based on genome information and biological information and behavior information, which are continuously collected, that are health information of the user on a display unit; and
displaying at least one of a target health state of the user and guidance information used for arriving at the target health state together with the future health risk.

41. A method of displaying health information using a health information display apparatus, the method comprising:
displaying a future health risk of a user, which is estimated based on genome information and biological information and behavior information, which are continuously collected, that are health information of the user on a display unit; and
displaying the health information of the user together with the future health risk.

42. A method of processing health information using a health information processing apparatus, the method comprising:
collecting genome information and biological information and behavior information being continuously collected, which are health information of each user, from each user;
storing the health information of each user that corresponds to a plurality of users;
deriving relevance between a combination of a type of genome derived from genome information and a type of lifestyle derived from biological information and behavior information and a predetermined purpose by receiving an input of the predetermined purpose and analyzing the stored health information corresponding to the plurality of users; and
outputting a result of the analysis.

43. A method of processing health information using a health information processing apparatus, the method comprising:
collecting genome information and biological information and behavior information being continuously collected, which are health information of each user, from each user;
storing the health information of each user that corresponds to a plurality of users;
receiving disclosure items of the health information and use permission of the health information for a disclosure destination from the user and outputting the health information according to a content of the received use permission; and
storing information of the use permission in accompaniment with the health information.

44. A method of processing health information using a health information processing apparatus, the method comprising:
collecting genome information and biological information being continuously collected, which are health information of each user, from each user;
storing the health information of each user that corresponds to a plurality of users;
comparing predetermined biological information with the biological information of each user, which is continuously collected, within a group formed by combining the genome information and attribute information of each user based on the stored information; and
representing a position of a value of the biological information of each user within the group based on a result of the comparison.

45. A method of processing health information using a health information processing apparatus, the method comprising:
collecting genome information and biological information being continuously collected, which are health information of each user, from each user;
storing the health information of each user that corresponds to a plurality of users;
integrating, for each group formed by combining the genome information and attribute information of each user, the biological information, which is continuously collected, of each user belonging to the group based on the stored information and comparing the biological information between the groups; and
representing a position of a value of the biological information of each group among the groups based on a result of the comparison.

46. A method of processing health information using a health information processing apparatus, the method comprising:
collecting genome information and biological information being continuously collected, which are health information of each user, from each user;
storing the health information of each user that corresponds to a plurality of users; and
deriving a result of a comparison between a reference value derived based on the genome information and a measured value collected from a user corresponding to the genome information for predetermined biological information.
